# FASCICULE DE BREVET EUROPEEN

(11) **EP 3 717 626 B1**
(45) Date de publication et mention de la délivrance du brevet: **15.12.2021**
(21) Numéro de dépôt: 18827198.5
(22) Date de dépôt: 29.11.2018
(51) Int. Cl.: C12M 1/12, C12M 1/34, C12Q 1/14, A61B 5/145, A61B 5/00, C12Q 1/04, C12Q 1/18, A61B 5/1477, A61B 5/1486, A61F 13/00

(54) **DISPOSITIFS MEDICAUX NON-IMPLANTABLES INTEGRANT UN DISPOSITIF DE DETECTION ET/OU D'IDENTIFICATION D'INFECTIONS MICROBIOLOGIQUES**
NICHTIMPLANTIERBARE MEDIZINISCHE VORRICHTUNGEN MIT EINER VORRICHTUNG ZUM NACHWEIS UND/ODER ZUR IDENTIFIZIERUNG MIKROBIOLOGISCHER INFEKTIONEN
NON-IMPLANTABLE MEDICAL DEVICES COMPRISING A DEVICE FOR DETECTING AND/OR IDENTIFYING MICROBIOLOGICAL INFECTIONS

(30) Priorité: 30.11.2017 FR 1761417
(43) Date de publication de la demande: 07.10.2020
(73) Titulaire: Biomérieux, 69280 Marcy-L'Etoile (FR); ELKEM SILICONES France SAS, 69003 Lyon (FR); F.D.S., 69730 Genay (FR); Fibroline, 69760 Limonest (FR); Hospices Civils De Lyon, 69002 Lyon (FR); Université Claude Bernard Lyon 1, 69100 Villeurbanne (FR); Institut National de la Santé Et de la Recherche Médicale, 75013 Paris (FR); Ecole Normale Supérieure de Lyon, 69007 Lyon (FR); Centre National de la Recherche Scientifique - CNRS, 75016 Paris (FR)
(72) Inventeur: ROZAND, Christine, 69290 St Genis les Ollieres (FR); DARDY, Aline, 69009 Lyon (FR); BERRAT, Fanny, 69160 Tassin la Demi-Lune (FR); MOINE, Caroline, 42290 Sorbiers (FR); SERRE, Philippe, 69660 Collonges au Mont d'Or (FR)
(74) Mandataire: Regimbeau
(86) Numéro de dépôt international: PCT/FR2018/053033
(87) Numéro de publication internationale: WO 2019/106296

(56) Documents cités:
- WO-A1-2010/022111
- WO-A2-2012/166848
- WO-A2-2017/168249
- US-A- 5 635 367
- US-A- 6 090 541

## Description

La présente invention appartient au domaine des dispositifs médicaux non-implantables utilisés pour recouvrir les plaies et les lésions cutanées en vue de les protéger des souillures et des infections extérieures, tels que pansements, compresses, bandages et autres articles analogues. La présente invention a plus précisément trait à des dispositifs médicaux non-implantables intégrant des moyens de diagnostic d'infections microbiologiques en vue de détecter la survenue d'une éventuelle infection.

Les plaies, quelle que soit leur gravité, sont soumises à des risques d'infection par des microorganismes pathogènes tels que bactéries, virus, champignons, protozoaires. Outre le fait d'interrompre le processus physiologique de cicatrisation, ces infections peuvent entrainer des complications sérieuses, telles que gangrène gazeuse, tétanos et à des incapacités à long terme (infection chronique de la plaie ou de l'os...), voire à un décès consécutif à un sepsis.

Dans le cas des hospitalisations, plus de 90 % des infections postopératoires constatées surviennent dans les 30 jours qui suivent un acte chirurgical. Les patients doivent ainsi être suivis au moins durant les 30 jours après avoir été opérés. Cliniquement, ce suivi consiste généralement en un examen visuel de la cicatrisation des plaies et la constatation d'un éventuel écoulement purulent qui -selon la couleur, la consistance et l'odeur- peut être le signe d'une infection. Lorsqu'une infection est suspectée, un prélèvement est réalisé puis expédié au laboratoire d'analyse pour des investigations complémentaires : analyses microscopiques, cultures microbiologiques et/ou antibiogrammes... En pratique, le praticien ne disposera des résultats d'analyses que le lendemain, au plus tôt.

Pour un meilleur suivi des patients, les professionnels de la santé (médecins, praticiens, infirmières...) ont émis le souhait de pouvoir disposer de tests et d'outils de diagnostic leur permettant de constater par eux-mêmes l'existence d'une infection et, éventuellement envisager le traitement à prescrire lors de consultation même.

Dans ce contexte, il a été envisagé d'intégrer des moyens de détection et/ou de diagnostic d'infections microbiologiques dans la structure des compresses, pansements, bandages et autres dispositifs médicaux non-implantables utilisés pour recouvrir et protéger les plaies.

Dans une première approche, sur la base des récentes avancées technologiques dans le domaine des microélectrodes, des capteurs électrochimiques ont été développés pour réaliser des analyses électrochimiques *in situ* de la composition chimique des exsudats des plaies et ce, en vue de détecter la présence de biomarqueurs associés à des infections microbiologiques.

Dans ce contexte, on peut notamment citer les travaux de Hajnsek et al., 2015 (SENSOR AND ACTUATORS B: CHEMICAL ; 265-274 : « An electrochemical sensorforfast detection of wound infection based on myeloperoxidase activity ») et Sismaet et al., 2016 (WOUND REPAIR AND REGENERATION; (24)366-372 : « Electrochemical detection of Pseudomonas in wound exudate samples from patients with chronic wounds »). Dans le premier exemple, le capteur permet la détection électrochimique d'une activité myéloperoxidase, qui est exprimée par de nombreuses bactéries pathogènes. Dans le deuxième exemple, le capteur permet une détection électrochimique de la pyocyanine, une molécule produite par *Pseudomonas aeruginosa.*

Ces capteurs électrochimiques sont aujourd'hui à un stade expérimental précoce de leur développement. Ils embarquent une technologie d'électrodes miniaturisées dont le coût actuel est incompatible avec le marché des pansements et des compresses. Par ailleurs, de tels capteurs électrochimiques ne sont pas autonomes. Pour fonctionner, ils doivent être connectés à tout un équipement externe apte à les alimenter en électricité, à recueillir les données transmises par les capteurs, et à les traiter avant de pouvoir rendre un résultat d'analyse au praticien ; autant d'équipements que ce dernier devra avoir à sa disposition.

Dans une deuxième approche, davantage en accord avec les contraintes technologiques et économiques du marché des pansements et des compresses, le concept d'un pansement intelligent (ou « *smart bandage* » en anglo-saxon) a été imaginé. Posé sur la plaie, celui-ci génèrerait un signal visible (apparition d'une couleur et/ou d'une fluorescence) en cas de survenue d'une infection microbiologique. Un tel pansement intelligent est connu par WO 2017/168249 A2.

Dans ce contexte, Brocklesby et al., 2013 (Medical hypotheses ; (80)237-240 : « Smart bandages - A colourful approach to early stage infection detection & control the wound care ») envisage la réalisation d'un pansement permettant la détection des bactéries productrices de *β*-lactamases. Pour ce faire, il est suggéré d'utiliser une céphalosporine « chimérique » chromogène, assemblée à partir d'un résidu de céphalosporine et d'un chromophore. Cette céphalosporine « chimérique » chromogène serait fixée grâce à un couplage chimique aux fibres du textile absorbant constitutif du pansement. Son hydrolyse par des céphalosporinases provoquerait la coloration du pansement. A ce jour, aucun dispositif ou pansement fonctionnant sur ce principe de détection n'a vu le jour.

Plus récemment, les chercheurs de l'Université de Bath au Royaume-Uni ont annoncé la mise au point d'un prototype de pansement intelligent émettant une fluorescence en réponse à une infection bactérienne (Thet et al., 2016 - ACS Applied Materials & Interfaces; 8(24): 14909-19 : « Prototype development of the intelligent hydrogel wound dressing and its efficacy in the detection of model pathogenic wound biofilm »)*.*

Ce pansement est formé d'une pièce de tissu absorbant, dont la face destinée à être appliquée au contact-même de la plaie du patient, est recouverte d'un hydrogel d'agarose 2 %. Dans la masse de cet hydrogel, se trouvent réparties des vésicules chargées de 5,6-carboflurescéine. La paroi de ces vésicules est constituée d'un assemblage de phospholipides, de cholestérol et de polymères de polyacétylène. Par sa composition, elle mime la membrane phospholipidique des cellules eucaryotes et est perforée par les cytotoxines que les bactéries pathogènes relarguent au cours d'une infection. La 5,6-carboflurescéine, quant à elle, est un composé qui présente deux particularités intéressantes, ingénieusement exploitées dans ce dispositif. La première est qu'elle s'auto-assemble en dimères, lorsque sa concentration est suffisamment élevée, puis se dissocie au fur et à mesure que sa concentration diminue. La deuxième particularité est qu'elle émet une fluorescence lorsqu'elle est à l'état monomérique ; à l'état de dimère, sa fluorescence est par contre bloquée.

En cas d'infection bactérienne, la paroi des vésicules est rompue par les cytotoxines relarguées, et la 5,6-carboflurescéine est libérée des vésicules et se dilue progressivement dans l'hydrogel. Exposé à une lumière UV, le pansement émet alors une fluorescence.

Actuellement, ce dispositif est au stade de prototype et de nombreux tests sont prévus pour s'assurer de l'innocuité de l'hydrogel d'agarose, de celle des vésicules et de la 5,6-carboflurescéine, qui se retrouveront directement au contact de la plaie du patient.

Un tel dispositif connait quelques inconvénients majeurs. Un premier inconvénient est lié à la non-spécificité de la détection. Un tel dispositif ne pourra qu'alerter le praticien sur la survenue d'une infection. Des tests complémentaires devront être réalisés, s'il souhaite connaître la nature du(es) pathogène(s) incriminé(s) et envisager la prescription d'un traitement spécifique. Un deuxième inconvénient vient de la stabilité dans le temps de l'hydrogel d'agarose et relève davantage des considérations propres à l'exploitation industrielle et commerciale d'un tel dispositif. Du fait de la présence de cet hydrogel, le succès commercial d'un tel dispositif pâtira d'une durée de péremption courte, d'importantes contraintes de conditionnement et de conservation.

La présente invention vise à résoudre les inconvénients précédemment énoncés. Elle a ainsi pour but général de proposer une solution technique à la réalisation de pansements dits intelligents, notamment en termes de simplicité de mise en œuvre et de spécificité de la détection.

Un objectif de la présente invention est de proposer un dispositif médical non implantable destiné à recouvrir les plaies et les lésions cutanées, de type pansement, compresse, bandage et autres articles absorbants, intégrant des moyens de diagnostic d'infections microbiologiques et permettant de signaler un état d'infection bactérienne par un changement de couleur et/ou par l'émission d'une fluorescence. Un autre objectif de la présente invention est de permettre une détection et une identification spécifique du(des) pathogène(s) en cause, grâce à l'apparition d'un profil de coloration et/ou d'émission de fluorescence, particuliers et caractérisables.

La présente invention a pour ambition de proposer des dispositifs médicaux non implantables destinés à recouvrir les plaies et les lésions cutanées, de type pansement, compresse, bandage, permettant le diagnostic *in situ* d'infections microbiologiques, et ce, de façon compatible avec les contraintes d'une exploitation industrielle et commerciale propres à ce secteur.

Enfin, la présente invention a pour vocation de proposer une technologie qui puisse être exploitée aussi bien pour la médecine humaine que pour la médecine vétérinaire.

Dans ce contexte, la présente invention a pour objet un dispositif médical non implantable destiné à recouvrir les plaies et les lésions cutanées, comprenant un biocapteur, caractérisé en ce que ledit biocapteur comprend une pièce en matériau absorbant et hydrophile fixant, en surface et/ou dans son épaisseur, une composition de poudres agglomérées comprenant des particules d'éthylène-acétate de vinyle (EVA) ayant une surface en partie recouverte au moins d'un sel d'acide orthophosphorique et d'un indicateur visuel de croissance microbiologique.

Le dispositif médical non-implantable est utilisé pour recouvrir les plaies et les lésions cutanées en vue de les protéger des souillures et des infections extérieures, et peut être un pansement, une compresse, un bandage et tout autre article absorbant adapté à un usage médical.

Le dispositif selon l'invention permet, de par la présence du biocapteur, de diagnostiquer *in situ* une infection microbiologique.

Afin d'accroitre la détection, le dispositif selon l'invention pourra comprendre des moyens permettant de diriger le sang ou l'exsudat d'une plaie vers le biocapteur.

Au sens de la présente invention, on utilisera le terme « liquide » pour désigner le sang ou l'exsudat d'une plaie ou au moins un élément constitutif dudit exsudat ou plus génériquement tout liquide aqueux biologique (ou « fluide biologique » ou « fluide corporel ») susceptibles de renfermer des microorganismes infectieux.

Ainsi, dans un mode de réalisation préféré, le dispositif médical non implantable comprend en outre une couche primaire adaptée pour diriger un liquide vers le biocapteur. Ladite couche primaire a avantageusement une zone préférentielle de collecte dudit liquide ainsi que des moyens de guidage dudit liquide en direction de ladite zone préférentielle de collecte.

Avantageusement, le dispositif médical non implantable comprend en outre une couche secondaire intégrant ledit biocapteur, la couche secondaire étant disposée par rapport à la couche primaire de sorte à ce que ledit biocapteur s'étend en regard de la zone préférentielle de collecte. La couche secondaire comprend avantageusement des première et deuxième zones, lesdites première et deuxième zones étant distinctes et présentant chacune un comportement respectif différent au contact dudit liquide.

Avantageusement, le dispositif médical non implantable comprend en outre une couche intermédiaire, qui est interposée entre lesdites couches primaire et secondaire. Avantageusement, la couche intermédiaire comprend elle-même au moins une première portion imperméable audit liquide et au moins une première portion perméable permettant le passage dudit liquide en provenance de la couche primaire vers la couche secondaire, ladite première portion imperméable s'étendant en regard de ladite première zone, la projection de la première portion imperméable dans un plan parallèle auxdites couches secondaire et intermédiaire recouvrant au moins la moitié de la surface de la projection de la première zone dans ce même plan, pour limiter le reflux dudit liquide depuis ladite première zone vers ladite couche primaire.

Avantageusement, le dispositif médical non implantable comprend en outre une couche barrière, recouvrant ladite couche secondaire et étant sensiblement imperméable audit liquide. Ladite couche barrière est avantageusement perméable à l'air, et de préférence transparente à la lumière.

Avantageusement, le dispositif médical non implantable comprend en outre une couche de transfert perméable audit liquide, disposée en regard de ladite couche primaire et permettant le passage dudit liquide en provenance du milieu extérieur audit dispositif médical vers ladite couche primaire.

Un tel dispositif médical multicouche permet de limiter le reflux du liquide à travers les différentes couches dudit dispositif médical, tout en étant de conception extrêmement simple, compacte et fiable.

Un dispositif médical multicouche est un assemblage avantageusement obtenu par empilement d'une pluralité de couches surfaciques distinctes les unes des autres, ces couches s'étendant de préférence selon des plans respectifs sensiblement parallèles les uns aux autres. Comme cela sera décrit plus en détail dans ce qui suit, les différentes couches qui composent un tel dispositif médical sont préférentiellement distinctes et de nature et fonction différentes, de sorte que ledit dispositif médical est avantageusement composite.

Le dispositif médical est avantageusement destiné à guider un liquide donné et conçu pour modifier la position d'au moins une partie dudit liquide avec lequel ledit dispositif médical est mis en contact. Plus spécifiquement, le dispositif médical multicouche est avantageusement conçu pour déplacer, acheminer, une certaine quantité dudit liquide depuis au moins une première région dudit dispositif médical vers au moins une seconde région de ce dernier, ladite seconde région étant de préférence distante et distincte de ladite première région et ladite seconde région intégrant le biocapteur. Ainsi que cela sera explicité ci-après, les différentes couches formant le dispositif médical multicouche de l'invention sont préférentiellement conçues, configurées et agencées l'une par rapport à l'autre pour permettre un déplacement du liquide considéré depuis ladite première région vers ladite deuxième région du dispositif médical, lesdites première et deuxième régions appartenant avantageusement à deux couches distinctes non immédiatement adjacentes. Le déplacement dudit liquide inclut, de préférence, au moins une composante orthogonale aux couches, voire même avantageusement une combinaison d'au moins une composante parallèle aux couches (c'est-à-dire un déplacement dans le plan d'extension d'au moins l'une des couches du dispositif médical) et d'au moins une composante orthogonale auxdites couches. En ce sens, le dispositif médical multicouche de l'invention constitue avantageusement un système fluidique ou a minima un composant de système fluidique.

Le dispositif médical de l'invention est avantageusement un pansement ou une compresse, plus avantageusement un pansement.

D'autres buts, caractéristiques et avantages de l'invention apparaîtront au vu de la description qui va suivre et des exemples développés ci-après, qui se réfèrent aux figures annexées et dans lesquelles :
- les figures 1 et 2 illustrent, de manière schématique , et respectivement selon une coupe verticale et selon une vue en perspective, un mode de réalisation préférentiel du dispositif médical multicouche de l'invention ;
- la figure 3 illustre, de manière schématique et en perspective, un autre mode de réalisation préférentiel du dispositif médical multicouche de l'invention ;
- la figure 4 illustre, de manière schématique et en vue de dessus, le recouvrement des projections orthogonales respectives dans un même plan fictif de certains éléments des couches du dispositif médical multicouche de l'invention ;
- les figures 5 et 6 illustrent de manière schématique, et respectivement selon une coupe verticale et selon une vue en perspective, une variante du mode de réalisation préférentiel des figures 1 et 2 ;
- la figure 7 illustre, de manière schématique et en perspective, une variante de la couche secondaire du dispositif médical selon l'invention, qui présente une ligne de compression ;
- la figure 8 est une représentation graphique de l'effet bactériostatique de l'EVA sur le développement des bactéries, et de l'inhibition de cet effet bactériostatique par les sels d'acide orthophosphorique ;
- la figure 9 correspond à deux photographies, prises en microscopie électronique à balayage, montrant une composition pulvérulente de particules dissociées ;
- la figure 10 correspond à deux photographies, prises en microscopie électronique à balayage, montrant une composition de poudres agglomérées comprenant des particules d'EVA ayant une surface en partie recouverte d'au moins un sel d'acide orthophosphorique et d'un indicateur visuel de croissance microbienne.

### Biocapteur

Le biocapteur comprend une pièce en matériau absorbant et hydrophile fixant, en surface et/ou dans son épaisseur, une composition de poudres agglomérées comprenant des particules d'éthylène-acétate de vinyle (ou EVA, pour « *ethylene-vinyl acetate* » en anglo-saxon) ayant une surface en partie recouverte au moins d'un sel d'acide orthophosphorique et d'un indicateur visuel de croissance microbiologique. En présence de microorganismes, en particulier de bactéries et de levures, ledit indicateur visuel de croissance microbiologique est apte à produire un signal visible à l'œil lorsque le biocapteur est exposé à la lumière blanche et/ou à une radiation UV.

Positionné à proximité d'une plaie ou d'une lésion, le dispositif médical est conçu pour pouvoir capter et recueillir l'exsudat des plaies, le guider vers le biocapteur et former un support propice à la croissance et au développement de microorganismes. La détection et/ou l'identification des microorganismes éventuellement présents s'effectuent grâce aux indicateurs visuels (chromogènes et/ou fluorogènes) de croissance microbiologique, sensibles à l'activité métabolique des microorganismes recherchés, présents dans le biocapteur.

Pour concevoir un tel biocapteur, les inventeurs ont mis à profit les techniques de microbiologie *in vitro* visant la détection et l'identification de microorganismes tels que des bactéries, levures, moisissures et amibes, et se sont plus particulièrement inspirés des travaux menés depuis les années 90 sur les milieux de culture chromogéniques et fluorogéniques (Perry et al., 2007 - Journal of Applied Microbiology ; 103:2046-55 : « The application ofchromogenic media in clinical microbiology »)*.*

De la composition de ces milieux de culture, les inventeurs ont essentiellement repris et appliqué les enseignements dispensés sur les indicateurs visuels de croissance microbiologique, dont l'utilisation permet une mise en évidence visuelle des microorganismes via leur activité métabolique. On trouve dans la littérature et dans le commerce, de nombreux indicateurs visuels de croissance microbiologique, susceptibles de pouvoir être utilisés pour confectionner un biocapteur selon l'invention. Il peut s'agir, par exemple, d'indicateurs colorés de pH, sensibles à la variation de pH induite par une hydrolyse de sucres ou d'autres substrats métabolisables par les microorganismes ciblés. Plus préférentiellement, il s'agit de substrats chromogènes et/ou fluorogènes aptes à libérer un composé chromophore et/ou fluorophore sous l'action d'une activité enzymatique particulière (telle que par exemple, les activités *β*-glucuronidase, *β*-galactosidase, *β-*glucosidase, *α*-glucuronidase, *α*-galactosidase, *α*-glucosidase, estérase, nitroréductase, phosphatases, aminopeptidase...).

Pour éliminer tout risque de surinfection de la plaie qui pourrait être imputable au dispositif médical, notamment au biocapteur, les inventeurs ont pris soin, dans la conception de ce dernier, de bannir tout ingrédient ayant un potentiel nutritif à l'égard des microorganismes. Avantageusement, la composition de poudres agglomérées fixée dans le biocapteur du dispositif médical selon l'invention ne présente aucune qualité nutritive notable/mesurable pour la croissance et le développement des microorganismes. En particulier, cette composition de poudres agglomérées est exempte de toute quantité physiologiquement effective de glucides, de lipides, d'acides aminés et de facteurs de croissance, apportés sous forme de compositions chimiquement bien définies ou sous la forme de compositions complexes telles que des mélanges de peptones, des extraits de levures, des sérums...

Ainsi, avec un dispositif médical selon l'invention, en cas d'infection microbiologique des plaies, les microorganismes ne peuvent puiser les nutriments nécessaires à leur développement que dans l'exsudat de la plaie, seule source nutritive disponible dans cet environnement particulier.

Pour embarquer et immobiliser dans le matériau absorbant du biocapteur une quantité d'indicateur visuel de croissance microbiologique qui soit opérante pour une détection et/ou une identification des microorganismes cibles, il a fallu chercher un adhésif pouvant répondre aux trois conditions majeures suivantes :
- être capable de fixer efficacement les indicateurs visuels de croissance microbiologique au matériau absorbant et hydrophile, sans nuire à leur fonctionnement ni gêner leur hydrolyse en cas d'infection par des microorganismes ciblés,
- être d'une efficacité suffisante pour que la quantité à utiliser ne soit pas dommageable pour les capacités d'absorption du matériau absorbant et hydrophile,
- être physiologiquement neutre à l'égard des microorganismes, c'est-à-dire ne présenter ni d'effets activateurs ou inhibiteurs notables sur la croissance et le développement des microorganismes.

Aucun des matériaux adhésifs testés par les inventeurs n'a pu satisfaire pleinement à ces trois conditions. Néanmoins, parmi les nombreux matériaux testés, l'EVA a suscité un grand intérêt auprès des inventeurs.

Résine synthétique bien connue depuis les années 50 pour ses propriétés thermoplastiques et thermodurcissables, l'EVA est préparé par copolymérisation de l'éthylène avec l'acétate de vinyle (la teneur pondérale d'acétate de vinyle varie généralement entre 10 et 40 %). On le retrouve sous des formes très diverses dans de nombreux secteurs de l'industrie (par exemple, un film plastique, un adhésif pour le thermo-scellage d'emballages, une colle dans la reliure des livres, une couche superficielle du papier glacé des magazines, un plastifiant dans les compositions de vernis et de peinture, un composant d'un matériau plastique thermo-moulé, un composant d'une composition d'encapsulation de principes actifs...). Dans le cadre de la mise au point des biocapteurs des dispositifs médicaux selon l'invention, les inventeurs ont pu constater une manipulation et une utilisation de l'EVA d'une grande simplicité. Un chauffage à des températures de l'ordre de 50-60°C permet de ramollir ces particules et rendre leur surface collante. Portées à de telles températures, les particules d'EVA présentent une bonne adhérence de surface pour un grand nombre de composés, en particulier pour les particules d'indicateurs visuels de croissance microbiologique qu'elles peuvent fixer et exposer à leur surface. Malheureusement, les inventeurs ont aussi constaté une activité bactériostatique de l'EVA, le rendant rédhibitoire pour une application dans le domaine de la microbiologie et d'autant plus, lorsque la finalité est une culture cellulaire à des fins de détection et/ou d'identification. Cependant, à force de persistance, les inventeurs sont parvenus à surmonter cet obstacle et ont démontré que l'adjonction d'un sel d'acide orthophosphorique permet d'inhiber cet effet bactériostatique indésirable.

Avantageusement et selon l'invention, le(les) sel(s) d'acide orthophosphorique utilisé(s) pour inhiber l'effet bactériostatique de l'EVA est(sont) choisi(s) parmi le dihydrogénophosphate de potassium (KH₂PO₄) et le dihydrogénophosphate de sodium (NaH₂PO₄).

Selon un premier mode de réalisation, le dihydrogénophosphate de potassium est utilisé pour inhiber l'effet bactériostatique de l'EVA. Le rapport pondéral KH₂PO₄/EVA est compris entre 1:25 et 1:8, préférentiellement compris entre 1:16 et 1:12.

Selon un deuxième mode de réalisation, le dihydrogénophosphate de sodium est utilisé pour inhiber l'effet bactériostatique de l'EVA. Le rapport pondéral NaH₂PO₄/EVA est compris entre 1:15 et 1:3, préférentiellement compris entre 1:10 et 1:5.

Selon un troisième mode de réalisation, pour inhiber l'effet bactériostatique de l'EVA, KH₂PO₄ et NH₂PO₄ sont utilisés concomitamment. Ainsi, KH₂PO₄ et NaH₂PO₄ sont concomitamment présents à la surface des particules d'EVA.

Avantageusement et selon l'invention, l'EVA présente une teneur pondérale en acétate de vinyle de 10 à 40 %, préférentiellement de l'ordre de 20 à 35 %.

Avantageusement et selon l'invention, la surface des particules d'EVA est également recouverte en partie d'un agent gélifiant qui, après absorption de l'exsudat, donne un gel. Utiliser un agent gélifiant dans ce contexte particulier présente plusieurs intérêts. En premier lieu, il améliore les performances d'absorption de la pièce en matériau absorbant et hydrophile, constitutive du biocapteur. Egalement, il permet de retenir et de bloquer en position les microorganismes captés.

Nombre d'agents gélifiants peuvent être utilisés à cette fin, notamment l'agar, l'agarose, la gomme de guar et la gomme de xanthane. La gomme de xanthane est préférée.

Selon un mode de réalisation particulier d'un biocapteur du dispositif médical selon l'invention, la surface des particules d'EVA peut aussi être en partie recouverte d'un agent sélectif. Cet agent sélectif est choisi pour bloquer ou ralentir le développement de la flore annexe, plutôt que celle d'un microorganisme-cible ou d'un groupe-cible de microorganismes. Il s'agit essentiellement de composés aux effets antibiotiques et/ou antifongiques, ayant une certaine spécificité de toxicité (toxicité plus faible pour les microorganismes-cibles que pour la flore annexe).

Selon un mode de réalisation particulier d'un biocapteur du dispositif médical selon l'invention, la surface des particules d'EVA peut aussi être en partie recouverte d'un agent stimulateur du métabolisme des bactéries, par exemple du chlorure de manganèse (MnCl₂).

Avantageusement et selon l'invention, les particules d'EVA présentent une granulométrie comprise entre 40 et 150 µm. Les particules de sel(s) d'acide orthophosphorique, les particules d'indicateur(s) visuel(s) de croissance microbiologique, éventuellement des particules d'agent gélifiant, des particules d'agent stimulateur du métabolisme des bactéries et des particules d'agent sélectif, qui recouvrent la surface de ces particules d'EVA ont une granulométrie avantageusement comprise entre 5 et 50 µm.

S'agissant de la pièce en matériau absorbant et hydrophile constitutive d'un biocapteur, de nombreux matériaux peuvent être utilisés. On utilise avantageusement un matériau fibreux, composé de fibres non-tissées, formant un ensemble ayant une intégrité et une cohérence mécanique. Lesdites fibres peuvent être des fibres cellulosiques naturelles (comme le coton) ou synthétiques (comme la rayonne), des fibres de cellulose modifiée (par exemple, la nitrocellulose), des fibres de polymères chimiques (tels que les copolymères acrylate/acrylamide).

Avantageusement, ladite pièce en matériau absorbant présente une masse surfacique comprise entre 50 g.m⁻² et 200 g.m⁻², pour une épaisseur avantageusement comprise entre 0,5 mm et 4 mm.

Selon un mode de réalisation préféré, cette pièce en matériau absorbant et hydrophile est réalisée à partir d'un textile non-tissé, par exemple à base de fibres de cellulose. Selon ce mode préféré, les particules d'EVA sont avantageusement fixées aux fibres du matériau, essentiellement dans son épaisseur.

### Couche primaire

Le dispositif médical comprend avantageusement en outre une couche primaire adaptée pour diriger un liquide vers le biocapteur.

Cette couche primaire permet ainsi de diriger un liquide vers au moins un point précis du dispositif médical où aura été positionné à cet effet un biocapteur, avantageusement de diriger ledit liquide en quantité suffisante et de la manière la plus rapide et précise possible vers au moins un point précis du dispositif médical où aura été positionné à cet effet un biocapteur.

La couche primaire du dispositif médical permet le guidage d'un liquide permettant de collecter des exsudats de la plaie ou au moins un élément constitutif desdits exsudats et de les acheminer rapidement et avec précision à proximité immédiate du biocapteur pour permettre le diagnostic et signaler un état d'infection bactérienne par un changement de couleur et/ou par l'émission d'une fluorescence.

La couche primaire permet de diriger le liquide à analyser en quantité suffisante et de la manière la plus rapide et précise possible vers au moins un point précis du dispositif médical où aura été positionné à cet effet un biocapteur. Ainsi, cette couche primaire permet de concentrer le liquide collecté au niveau de la plaie en au moins un point très précis du dispositif médical.

La couche primaire permet un acheminement rapide et précis dudit liquide vers une zone cible précise prédéfinie de ce dernier ou vers une pluralité de zones cibles précises prédéfinie de ce dernier, distinctes et distantes, tout en étant de conception extrêmement simple, compacte et fiable.

La couche primaire présente en outre un effet anti-reflux particulièrement efficace.

La couche primaire est particulièrement facile à concevoir et à fabriquer à l'aide de moyens techniques classiques.

Selon l'invention, le dispositif médical 1, l' comprend avantageusement au moins une couche primaire 2, 2' destinée à venir en contact avec un liquide. De préférence, cette couche primaire 2, 2' est destinée à venir prioritairement en contact avec ledit liquide, c'est-à-dire qu'elle constitue avantageusement une couche dudit dispositif médical 1, l' qui est destinée à venir en contact avec le liquide avant toutes ou partie des autres couches dudit dispositif médical 1, 1', ledit liquide pénétrant ainsi avantageusement ledit dispositif médical 1, 1', depuis le milieu extérieur à ce dernier, par l'intermédiaire de ladite couche primaire 2, 2'. De préférence, ladite couche primaire 2, 2' constitue l'une des couches les plus superficielles du dispositif médical 1, 1'. Cette couche primaire 2, 2' pourra ainsi avantageusement correspondre à ladite première région du dispositif médical 1,1' évoquée ci-dessus.

De préférence, cette couche primaire 2, 2' est formée d'une pièce primaire 2A, 2A' de textile, lequel est préférentiellement non-tissé, poreuse et/ou hydrophile (par exemple en papier ou coton). Ladite pièce primaire 2A, 2A' de textile est avantageusement drainante, c'est-à-dire que si ladite pièce primaire 2A, 2A' peut éventuellement absorber ledit liquide du fait de son caractère poreux et/ou hydrophile, elle est cependant avantageusement sensiblement dépourvue de propriété de rétention dudit liquide et elle est, au contraire, préférentiellement dotée d'une grande capacité de diffusion dudit liquide en son sein. De préférence encore, l'épaisseur de la couche de primaire 2, 2' est sensiblement comprise entre 0,1 et 0,5 mm.

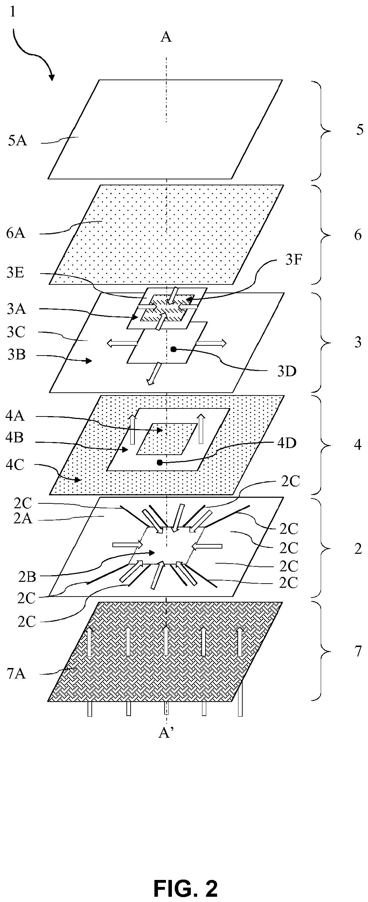

De manière avantageuse, ladite couche primaire 2, 2' comprend au moins une zone préférentielle de collecte 2B, 2B', 2B" (identifiée(s) en pointillés aux figures 2, 3 et 6) dudit liquide, ainsi que des moyens de guidage 2C, 2C' dudit liquide en direction de ladite zone de collecte 2B, 2B', 2B". Ladite zone préférentielle de collecte 2B, 2B', 2B" correspond ainsi avantageusement à une zone particulière prédéfinie de ladite couche primaire 2, 2' vers laquelle ledit liquide pénétrant dans le dispositif médical 1, l' est préférentiellement dirigé, avantageusement selon un déplacement dudit liquide parallèle au plan d'extension primaire de ladite couche primaire 2, 2'. De préférence, comme illustré aux figures, ladite zone préférentielle de collecte 2B, 2B', 2B" est sensiblement localisée à distance des bords primaires qui définissent le contour primaire de ladite couche primaire 2, 2', de sorte à limiter le risque de fuite dudit liquide hors de la couche primaire 2, 2' par les bords primaires de cette dernière. Avantageusement, la couche primaire 2, 2' présentant une première superficie SI (aire), la zone préférentielle de collecte 2B, 2B', 2B" présente quant à elle une deuxième superficie S2 qui sensiblement inférieure à ladite première superficie SI de la couche primaire 2, 2'. Ainsi, ladite zone préférentielle de collecte 2B, 2B', 2B" pourra être définie de sorte que ladite deuxième superficie S2 soit préférentiellement égale ou inférieure à la moitié, au tiers, au quart, au sixième, voire au dixième de ladite première superficie SI de la couche primaire 2, 2', selon notamment l'application visée et la quantité dudit liquide que le dispositif fluidique est destiné à manipuler.

Il est à noter que, si ladite zone préférentielle de collecte 2B, 2B', 2B"est représentée aux figures selon une forme rectangulaire, cette forme est purement schématique et n'est donnée qu'à titre illustratif uniquement, et pourra bien évidemment affecter toute autre forme souhaitée (circulaire, oblongue, etc.).

De préférence, allongés, et de préférence sensiblement rectilignes et continus, lesdits moyens de guidage 2C, 2C' sont distincts les unes des autres, c'est-à-dire qu'ils sont disposés à distance les uns des autres et ne se touchent ou se recoupent pas. En outre, et de manière particulièrement intéressante, lesdits moyens de guidage 2C, 2C' sont orientés selon des directions d'extension longitudinale (moyenne) sécantes en un point (fictif, non représenté) situé dans (ou au moins à proximité immédiate de) ladite zone préférentielle de collecte 2B, 2B', 2B", pour définir entre elles au moins un (et de préférence une pluralité de) canal primaire (représenté par une flèche sur les figures) de guidage dudit liquide qui converge en direction de ladite zone préférentielle de collecte 2B, 2B', 2B".

De préférence, lesdits moyens de guidage 2C, 2C' sont agencés autour de la zone préférentielle de collecte 2B, 2B', 2B". De manière préférentielle, et tel que cela est particulièrement visible aux figures 2, 3 et 6, les éléments barrières 2C, 2C' sont agencés pour former deux à deux des « canaux » convergents qui dirigent (c'est-à-dire « forcent » le déplacement) ledit liquide présent dans la couche primaire 2, 2' vers ladite zone préférentielle de collecte 2B, 2B', 2B". De préférence, lesdits moyens de guidage 2C, 2C' (en l'espèce, de préférence, lesdits éléments barrières 2C, 2C') sont agencés autour de la zone préférentielle de collecte 2B, 2B', 2B". De préférence encore, ils sont agencés sensiblement sur tout le contour de la zone préférentielle de collecte 2B, 2B', 2B", de sorte à entourer cette dernière. Avantageusement positionnés l'un par rapport à l'autre de manière sensiblement régulière, voire équi-répartis, autour de la zone préférentielle de collecte 2B, 2B', 2B", les moyens de guidage 2C, 2C' définissent ainsi de préférence un schéma « en étoile » ou « en rayons » sensiblement centré sur la zone préférentielle de collecte 2B, 2B', 2B".

De manière avantageuse, l'extrémité proximale primaire respective de chaque moyens de guidage 2C, 2C' est en contact avec le contour primaire de ladite zone préférentielle de collecte 2B, 2B', 2B", lesdits moyens de guidage 2C, 2C' ne se prolongeant pas, de préférence, au sein de la zone préférentielle de collecte 2B, 2B', 2B"en elle-même.

Il a été observé qu'une telle configuration des moyens de guidage 2C, 2C' et de la zone préférentielle de collecte 2B, 2B', 2B" permet de créer un phénomène de drainage guidé particulièrement efficace du liquide en contact avec ladite couche primaire 2, 2' en direction de ladite zone préférentielle de collecte 2B, 2B', 2B", selon une direction sensiblement parallèle au premier plan P1 d'extension moyenne de la nappe 2. En outre, une telle configuration s'avère également particulièrement efficace pour limiter très fortement tout risque de reflux dudit liquide depuis la zone préférentielle de collecte 2B, 2B', 2B" vers le ou les bords périphériques.

Lesdits moyens de guidage 2C, 2C' sont disposés dans au moins une partie de l'épaisseur, et de préférence dans toute l'épaisseur, de ladite couche primaire 2, 2'. De la sorte, ledit au moins un canal primaire est avantageusement défini entre deux moyens de guidage 2C, 2C' et dans toute l'épaisseur de couche primaire 2, 2'. On évite ainsi tout risque de fuite et de diffusion, dans l'épaisseur de la couche primaire 2, 2', du liquide circulant dans un canal primaire.

Pour garantir un guidage le plus efficace possible dudit liquide, lesdits moyens de guidage 2C, 2C' sont de préférence sensiblement imperméables audit liquide, et constituent donc chacun un obstacle sensiblement étanche s'opposant au passage dudit liquide à travers lui. Une telle imperméabilité peut être obtenue de manière physique (moyens de guidage 2C, 2C' en un matériau non poreux en regard du liquide considéré) ou chimique (moyens de guidage 2C, 2C' en un matériau par exemple insoluble dans ledit liquide considéré).

Lesdits moyens de guidage 2C, 2C' sont de préférence en matériau polymère hydrophobe, et de préférence encore non poreuses vis-à-vis du liquide. Un tel matériau polymère hydrophobe peut, par exemple, être choisi dans le groupe comprenant : un silicone, un polyuréthane, un polyéthylène, un polyamide et un polyester. Avantageusement, ledit matériau polymère hydrophobe pourra être un matériau polymère thermoplastique, de préférence une colle thermofusible de type adhésif sensible à la pression (PSA pour « *pressure sensitive adhesive* » en anglo-saxon).

Comme cela sera explicité plus loin, de tels moyens de guidage 2C, 2C' peuvent, par exemple, être réalisés par enduction et imprégnation locale de ladite pièce primaire 2A, 2A' de textile à l'aide dudit matériau polymère hydrophobe. Bien évidemment, lesdits moyens de guidage 2C, 2C' pourront être constitués de manière différente, sans pour autant sortir du cadre de l'invention.

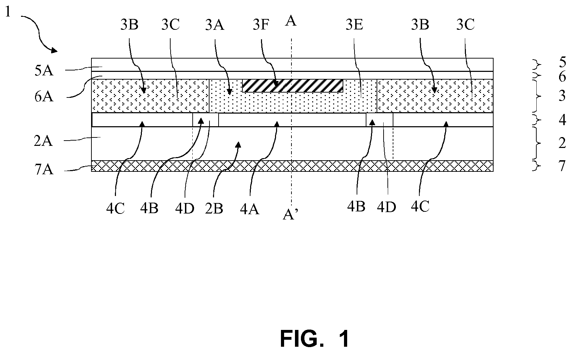

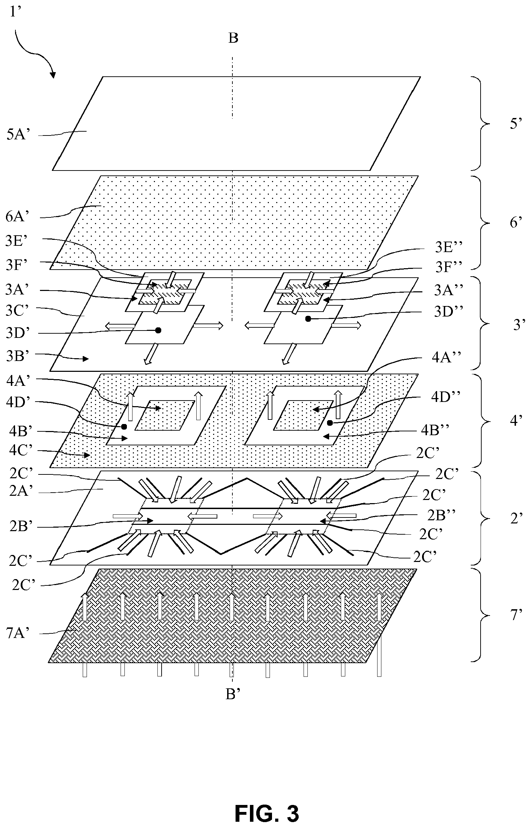

Selon un premier mode de réalisation préférentiel illustré aux figures 1, 2, 5 et 6, la couche primaire 2 comprend une seule zone préférentielle de collecte 2B. Cependant, ladite couche primaire 2' pourra comprendre plusieurs zones préférentielles de collecte distinctes. Ainsi, selon un deuxième mode de réalisation préférentiel, illustré à la figure 3, la couche primaire 2' comprend préférentiellement une première 2B' et une deuxième 2B" zones préférentielles de collecte, distinctes et préférentiellement distantes l'une de l'autre. Avantageusement, lesdits moyens de guidage 2C' seront alors agencés selon une pluralité de schémas « en étoile » ou « en rayons » respectivement centrés sur chacune desdites première 2B' et deuxième 2B" zones préférentielles de collecte. En outre, afin de favoriser un guidage sensiblement simultané et homogène dudit liquide en direction de chacune des zones préférentielles de collecte 2B', 2B", la couche primaire 2' pourra en outre avantageusement comprendre une pluralité de barrières supplémentaires de guidage dudit liquide, agencées de manière sensiblement parallèle et définissant au moins un canal supplémentaire de guidage dudit liquide qui relie entre elles lesdites zones préférentielles de collecte 2B', 2B" (figure 3).

De manière avantageuse, tel qu'illustré à la figure 3, ladite zone préférentielle de collecte 2B' et ladite zone préférentielle de collecte 2B" sont avantageusement identiques entre elles et positionnées en miroir de part et d'autre du plan orthogonal au plan d'extension moyen de la couche primaire 2'. La zone préférentielle de collecte 2B' et les moyens de guidage 2C' définissent un « motif fluidique » qui est avantageusement reproduit à l'identique par symétrie. Alternativement, on pourrait bien entendu envisager, sans sortir pour autant du cadre de l'invention, que la zone préférentielle de collecte 2B' présente une forme et/ou des dimensions tout à fait différente(s) de celle(s) de la zone préférentielle de collecte 2B", et/ou que la conformation et la configuration des moyens de guidage 2C' diffèrent d'un motif fluidique à l'autre.

Afin de favoriser un guidage sensiblement simultané et homogène dudit liquide en direction de chacune desdites zones préférentielles de collecte 2B' et 2B", la couche primaire 2' peut en outre avantageusement comprendre une pluralité de moyens de guidage 2D' lesquels sont sensiblement parallèles entre eux et définissent un canal secondaire de guidage dudit liquide qui relie entre elles lesdites zones préférentielles de collecte 2B' et 2B" (figure 3).

De préférence sensiblement rectilignes et continus, ces moyens de guidage 2D' sont, à l'instar desdits moyens de guidage 2C', distincts les unes des autres, c'est-à-dire qu'ils sont disposés à distance les uns des autres et ne se touchent pas. Avantageusement, chacun desdits moyens de guidage 2D' s'étend selon une direction d'extension longitudinale moyenne sensiblement parallèle au plan d'extension moyenne de la couche primaire 2'. Avantageusement encore, ces moyens de guidage 2D' sont sensiblement imperméables audit liquide, et de préférence en un matériau identique à celui desdits moyens de guidage 2C'. En outre, lesdits moyens de guidage 2D' pénètrent préférentiellement dans toute l'épaisseur de la couche primaire 2', et sont d'une largeur sensiblement voisines (sinon identiques) de celle desdits moyens de guidage 2C'.

De manière particulièrement intéressante, et afin d'améliorer encore la simultanéité et l'homogénéité du phénomène de guidage dudit liquide vers chacune des zones préférentielles de collecte 2B' et 2B", lesdits moyens de guidage 2D' traversent avantageusement de part en part lesdites zones préférentielles de collecte 2B' et 2B", chacun moyens de guidage 2D' reliant l'un des moyens de guidage 2C' adjacent à la zone préférentielle de collecte 2B' à l'un des moyens de guidage 2C' adjacent à la zone préférentielle de collecte 2B". Autrement dit, et tel qu'illustré à la figure 3, les barrières moyens de guidage 2D' sont préférentiellement agencées parallèlement l'un à l'autre pour former un canal secondaire continu qui traverse chacune des zones préférentielles de collecte 2B' et 2B", en reliant deux moyens de guidage 2C'. On obtient avantageusement de la sorte, tel que cela ressort de la figure 3, un canal secondaire qui se prolonge en chacune de ses extrémités par un canal primaire.

Lesdites zones préférentielles de collecte 2B' et 2B" étant distinctes et préférentiellement positionnées à distance l'une de l'autre selon le plan d'extension moyenne de la couche primaire 2', ladite couche primaire 2' comporte de préférence une région (ou une étendue) surfacique de la couche primaire 2' qui s'étend entre lesdites zones préférentielles de collecte 2B' et 2B". Séparant ces dernières, ladite région surfacique relie avantageusement les portions des contours primaires respectifs desdites zones préférentielles de collecte 2B' et 2B".

A ce titre, et pour limiter voire interdire le déplacement (ou reflux) dudit liquide depuis ladite région surfacique en direction d'au moins un des bords périphériques, ladite couche primaire 2' comprend avantageusement une pluralité de moyens de guidage tertiaires 2E', chacun étant respectivement interposé entre ladite région surfacique et au moins un des bords périphériques de la couche primaire 2'. Lesdits moyens de guidage 2E' sont avantageusement agencés pour s'opposer au déplacement dudit liquide, depuis ladite région surfacique vers au moins un des bords périphériques, via la partie du contour de ladite région non bordé par les zones préférentielles de collecte 2B' et 2B".

De préférence, afin de simplifier au maximum la conception et la fabrication de ladite couche primaire 2', ces moyens de guidage 2E' sont respectivement formées de la réunion de deux moyens de guidage 2C', lesquels sont préférentiellement identiques et symétrique selon le plan B-B'.

De manière plus générale, d'autres conformations et configurations desdits moyens de guidage 2C, 2C' (et, optionnellement 2D' et/ou 2E') pourront être mis en œuvre sans pour autant sortir du cadre de l'invention, dès lors que deux moyens adjacents ne définissent pas entre eux un canal de guidage du liquide qui serait convergent en direction d'au moins un des bords périphériques de la couche primaire 2, 2'.

De manière préférentielle, ladite pièce primaire 2A, 2A' de textile, d'un seul tenant, est quant à elle préférentiellement constituée à 80 % de pulpe de bois (cellulose) et à 20 % de fibres de polyester (par exemple du PET), et présente préférentiellement une masse surfacique sensiblement égale à 28 g.m⁻². Une telle pièce primaire 2A, 2A' de textile peut, par exemple, être obtenue par voie humide ou papetière (procédé « *wet laid* » en anglo-saxon), par incorporation de fibres de polyester dans une pâte cellulosique de pulpe de bois.

### Couche secondaire

Tel qu'illustré aux figures, dans un mode de réalisation avantageux, le dispositif médical 1, l' de l'invention comprend également au moins une couche secondaire 3, 3', distincte de ladite couche primaire 2, 2', intégrant ledit biocapteur 3E, 3E', 3E", la couche secondaire étant disposée par rapport à la couche primaire de sorte à ce que ledit biocapteur s'étend en regard de la zone préférentielle de collecte.

De préférence agencée en regard (avantageusement à l'aplomb) de ladite couche primaire 2, 2', ladite couche secondaire 3, 3' s'étend préférentiellement selon un plan d'extension secondaire sensiblement parallèle au plan d'extension primaire de ladite couche primaire 2, 2'. Cette couche secondaire 3, 3' correspond avantageusement à ladite deuxième région du dispositif médical 1,1' évoquée ci-dessus, vers laquelle ledit liquide, en provenance de ladite couche primaire 2, 2', est dirigé et acheminé. Si l'on se réfère aux figures, l'aplomb est avantageusement donné par la direction de l'axe A-A', B-B', sensiblement orthogonal aux plans d'extension respectifs des différentes couches dudit dispositif médical 1, 1'. Dans les modes de réalisations préférentiels illustrés auxdites figures, ladite couche secondaire 3, 3' est avantageusement agencée en regard, à l'aplomb et au-dessus de ladite couche primaire 2, 2'. De préférence, ladite couche secondaire 3, 3' est absorbante vis-à-vis dudit liquide, c'est-à-dire que le ou les matériaux qui la composent sont avantageusement pourvus d'une capacité d'absorption dudit liquide non nulle, et de manière encore plus avantageuse, d'une capacité de rétention significative dudit liquide. Selon l'invention, cette couche secondaire 3, 3' ne forme pas une couche parfaitement et strictement homogène en tout point. En effet, ladite couche secondaire 3, 3' comprend elle-même au moins des première 3A, 3A' et deuxième zones 3B, 3B', lesquelles première 3A, 3A' et deuxième 3B, 3B' zones sont distinctes l'une de l'autre et présentent chacune un comportement respectif différent au contact dudit liquide.

De manière particulièrement intéressante, ces première 3A, 3A' et deuxième 3B, 3B' zones sont ainsi prévues et conçues pour réagir différemment, d'un point de vue physique et/ou chimique, en présence du même dit liquide avec lequel elles sont mises en contact. Dès lors, lesdites première 3A, 3A' et deuxième 3B, 3B' zones constituent avantageusement des zones fonctionnelles différentes en regard du guidage dudit liquide au sein du dispositif médical 1, 1'. En particulier, ladite première zone 3A, 3A' correspond préférentiellement à une zone de la couche secondaire 3, 3' vers laquelle ledit liquide, en provenance de ladite couche primaire 2, 2', sera prioritairement déplacé, dirigé, tandis que ladite deuxième zone 3B, 3B' correspond avantageusement de préférence à une autre zone de la couche secondaire 3, 3' vers laquelle ledit liquide sera également dirigé, mais de manière cependant moins prioritaire. En ce sens, ladite première zone 3A, 3A' constitue avantageusement une première zone « cible » d'intérêt pour ledit liquide.

A ce titre, ladite première zone 3A, 3A' présente avantageusement une première vitesse d'absorption et une première capacité d'absorption dudit liquide, tandis ladite deuxième zone 3B, 3B' présente une deuxième vitesse d'absorption et une deuxième capacité d'absorption, ladite première vitesse d'absorption étant strictement supérieure à ladite deuxième vitesse d'absorption. Ladite première capacité d'absorption est quant à elle de préférence strictement inférieure à ladite deuxième capacité d'absorption. On entend ici préférentiellement par « capacité d'absorption » le volume maximal de liquide que peut absorber en son sein, et avantageusement retenir, le matériau formant la zone 3A, 3B, 3A', 3B' considérée de ladite couche secondaire 3, 3'. Le terme « vitesse d'absorption » se réfère quant à lui préférentiellement à la rapidité à laquelle le matériau formant la zone 3A, 3B, 3A', 3B' considérée est susceptible d'absorber une quantité donnée dudit liquide. Une telle différenciation de vitesse et de capacité d'absorption entre lesdites première 3A, 3A' et deuxième 3B, 3B' zones peut, par exemple, être conférée à la couche secondaire 3, 3' par une modification localisée (par exemple par compression) de la densité du matériau formant ladite couche secondaire 3, 3' ou par l'emploi, au sein de la même dite couche secondaire 3, 3', de matériaux de nature et propriétés différentes. Ces matériaux seront choisis de manière adéquate en regard des propriétés d'absorption recherchées. De la sorte, il est ainsi possible de générer un phénomène compétitif entre lesdites première 3A, 3A' et deuxième 3B, 3B' zones en regard dudit liquide lorsque ce dernier entre en contact avec la couche secondaire 3, 3'. En effet, ladite première zone 3A, 3A' « attirera » prioritairement ledit liquide vers elle, du fait de sa plus grande vitesse d'absorption. Dans un premier temps, la quasi-totalité (ou au moins la majorité en volume) du liquide atteignant la couche secondaire 3, 3' sera donc avantageusement dirigée vers la première zone 3A, 3A' et absorbée par cette dernière. Puis, une fois atteinte la première capacité d'absorption de ladite première zone 3A, 3A', c'est-à-dire une fois que cette dernière est saturée en liquide (ce qui peut par exemple correspondre à un volume de liquide sensiblement proche de 1 ml), le liquide continuant à affluer vers la couche secondaire 3, 3' sera alors « attiré » majoritairement (en volume) vers ladite deuxième zone 3B, 3B', du fait cette fois de la plus grande capacité d'absorption de cette dernière. Bien évidemment, lesdites première 3A, 3A' et deuxième zones 3B, 3B' pourront présenter des comportements respectifs qui différent de ceux décrits ci-dessus, sans pour autant sortir du cadre de l'invention.

Avantageusement, la deuxième zone 3B, 3B' de la couche secondaire 3, 3' pourra être imprégnée d'une substance hydrophobe, telle que par exemple de l'EVA. Ceci permet d'abaisser davantage ladite deuxième vitesse d'absorption de la deuxième zone 3B, 3B' et favorise l'accumulation dudit liquide dans lesdites première 3A, 3A' zones plutôt que dans lesdites deuxième 3B, 3B' zones.

De manière encore plus préférentielle, pour faciliter et accélérer le déplacement dudit liquide depuis la couche primaire 2, 2' vers lesdites première 3A, 3A' et deuxième 3B, 3B' zones de la couche secondaire 3, 3', ladite couche primaire 2, 2' présente avantageusement une troisième capacité d'absorption dudit liquide, qui est strictement inférieure, et de préférence très inférieure, auxdites première et deuxième capacités d'absorption des première 3A, 3A' et deuxième 3B, 3B' zones de la couche secondaire 3, 3'. De préférence encore, ladite couche primaire 2, 2' présente avantageusement une troisième vitesse d'absorption dudit liquide, qui est strictement supérieure, et de préférence très supérieure, auxdites première et deuxième vitesses d'absorption des première 3A, 3A' et deuxième 3B, 3B' zones de la couche secondaire 3, 3'.

Ladite première zone 3A, 3A' de la couche secondaire 3, 3' comporte un biocapteur 3E, 3E', tel que décrit précédemment, comprenant une composition de poudres agglomérées, telle que décrite précédemment, comprenant des particules d'EVA ayant une surface en partie recouverte au moins d'un sel d'acide orthophosphorique et d'un indicateur visuel de croissance microbiologique.

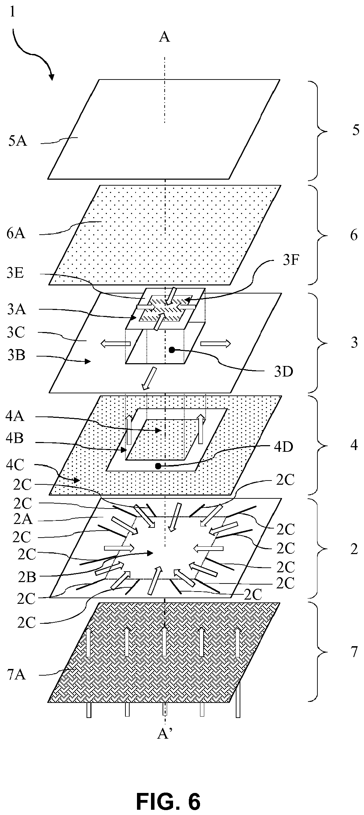

Selon le premier mode de réalisation préférentiel illustré aux figures 2 et 6, la couche secondaire 3 du dispositif médical 1 comprend une première zone 3A et une deuxième zone 3B conformes à ce qui précède. De préférence, tel qu'illustré aux figures, ladite première zone 3A est délimitée par un contour externe fermé et est entourée de manière contiguë par ladite deuxième zone 3B. Autrement dit, ladite deuxième zone 3B jouxte immédiatement ladite première zone 3A, bordant cette dernière sur toute sa périphérie. Cependant, ladite couche secondaire pourra comprendre davantage de zones de comportements différents, sans sortir pour autant du cadre de l'invention. Ainsi, selon le deuxième mode de réalisation préférentiel, illustré à la figure 3, la couche secondaire 3' du dispositif médical 1' comprend avantageusement une première zone 3A' et une deuxième zone 3B', conformes à ce qui précède, ainsi qu'une troisième zone 3A". Cette troisième zone 3A", avantageusement distinctes desdites première zone 3A' et deuxième zone 3B', peut elle aussi être délimitée par un contour externe fermé, et elle est de préférence entourée de manière contiguë par ladite deuxième zone 3B'. Ladite troisième zone 3A" présente, de préférence, des propriétés et comportement identiques à ceux de ladite première zone 3A', en particulier en termes de vitesse et de capacité d'absorption. Néanmoins, il est tout à fait envisageable, sans sortir pour autant du cadre de l'invention, que lesdites première 3A', deuxième 3B' et troisième 3A" zones de la couche secondaire 3' chacune un comportement respectif différent au contact dudit liquide. Ladite troisième zone 3A" constitue ainsi avantageusement une deuxième zone « cible » de ladite couche secondaire 3'. Ladite troisième zone 3A" de la couche secondaire 3' comporte un biocapteur 3E", tel que décrit précédemment, comprenant une composition de poudres agglomérées, telle que décrite précédemment comprenant des particules d'EVA ayant une surface en partie recouverte au moins d'un sel d'acide orthophosphorique et d'un indicateur visuel de croissance microbiologique. Les zones 3A', 3A" pourront comprendre le même biocapteur, ou des biocapteurs correspondant à des variantes différentes de l'invention, notamment comprenant des compositions de poudres agglomérées différentes pouvant comprendre des indicateurs visuels de croissance microbiologique différents.

De préférence, l'épaisseur de la couche secondaire 3, 3' est sensiblement comprise entre 2 et 5 mm. Avantageusement, tel qu'illustré aux figures 2, 3 et 6, ladite couche secondaire 3, 3' n'est pas formée d'un seul tenant, c'est-à-dire d'une seule et même pièce surfacique, mais consiste au contraire en un assemblage surfacique de pièces surfaciques distinctes. Tel qu'illustré aux figures 2 et 6, la couche secondaire 3 est ainsi préférentiellement formée au moins :
- d'une pièce secondaire 3C de textile pourvue d'au moins une première fenêtre 3D traversante ménagée à travers ladite pièce secondaire 3C, ladite pièce secondaire 3C formant (ou contribuant au moins à former) ladite deuxième zone 3B de la couche secondaire 3, et
- d'une première pièce tertiaire 3E de textile, distincte de ladite pièce secondaire 3C et de forme sensiblement complémentaire à ladite première fenêtre 3D, ladite première pièce tertiaire 3E étant logée étroitement (c'est-à-dire de préférence « bords à bords ») au sein de ladite première fenêtre 3D et formant (ou contribuant au moins à former) ladite première zone 3A de la couche secondaire. Cette première pièce tertiaire 3E de textile est le biocapteur décrit précédemment ou comprend le biocapteur décrit précédemment.

Alternativement, dans le deuxième mode de réalisation préférentiel illustré à la figure 3, la couche secondaire 3' pourra avantageusement être formée au moins :
- d'une pièce secondaire 3C' de textile pourvue d'une première 3D' et d'une deuxième 3D" fenêtres traversantes ménagées à travers ladite pièce secondaire 3C' et avantageusement distinctes et distantes l'une de l'autre,
- et d'une première 3E' et d'une deuxième 3E" pièces tertiaires de textile, distinctes l'une de l'autre et de ladite pièce secondaire 3C', et respectivement de forme sensiblement complémentaire à ladite première 3D' et deuxième 3D" fenêtres, ces première 3E' et deuxième 3E" pièces tertiaires étant respectivement logées étroitement au sein desdites première 3D' et deuxième 3D" fenêtre 3D et formant (ou contribuant au moins à former) respectivement lesdites première 3A et troisième 3A" zones de la couche secondaire 3'.

Lesdites première 3D' et deuxième 3D" fenêtres, d'une part, et lesdites première 3E' et deuxième 3E" pièces tertiaires d'autre part, sont de préférence identiques, mais pourraient bien évidemment être différentes, sans pour autant sortir du cadre de l'invention.

Lesdites première 3E' et deuxième 3E" pièces tertiaires sont un biocapteur tel que décrit précédemment ou comprennent un biocapteur tel que décrit précédemment.

Se présentant avantageusement sous la forme de nappes surfaciques et préférentiellement choisies sensiblement de même épaisseur, lesdites pièces secondaire 3C, 3C' et tertiaire 3E, 3E', 3E" de textile sont donc agencées de manière coplanaire pour former (ou au moins contribuer à former) ladite couche secondaire 3, 3'. Ladite première fenêtre 3D (respectivement lesdites première 3D' et deuxième 3D" fenêtres) et ladite première pièce tertiaire 3E (respectivement lesdites première 3E' et deuxième 3E" pièces tertiaires) correspondante peuvent affecter respectivement toutes formes complémentaires connues et, par exemple, affecter respectivement une forme rectangulaire (tel qu'illustré aux figures 2, 3 et 6) ou en encore circulaire. Ladite couche secondaire 3, 3' et ses première 3A, 3A' et deuxième 3B, 3B' zones (voire troisième zone 3A") sont ainsi relativement simples à concevoir et à fabriquer, le choix des matériaux formant respectivement lesdites première 3A, 3A' et deuxième 3B, 3B' (voire troisième zone 3A") zones étant dès lors très vaste, puisque ladite couche secondaire 3, 3' n'est pas formée d'un seul tenant.

De préférence, ladite pièce secondaire 3C, 3C' de textile est une pièce de textile préférentiellement non-tissé et hydrophile et, de manière encore plus préférentielle, aiguilleté. L'aiguilletage du textile permet en effet avantageusement d'« aérer » le textile et, dans une certaine mesure, d'augmenter la capacité d'absorption de ladite pièce secondaire 3C, 3C' (et donc d'augmenter ladite deuxième capacité d'absorption de ladite deuxième zone 3B, 3B'). De manière plus préférentielle encore, ladite pièce secondaire 3C, 3C' de textile est constituée à 70 % de fibres de polyéthylène et à 30 % de fibres de polypropylène, et présente préférentiellement une masse surfacique (« grammage ») sensiblement égale à 220 g.m^{―2}.

Ladite première pièce tertiaire 3E (respectivement lesdites première 3E' et deuxième 3E" pièces tertiaires), est quant à elle préférentiellement une pièce en matériau absorbant et hydrophile fixant, en surface et/ou dans son épaisseur, une composition de poudres agglomérées comprenant des particules d'éthylène-acétate de vinyle ayant une surface en partie recouverte au moins d'un sel d'acide orthophosphorique et d'un indicateur visuel de croissance microbiologique. Ladite première pièce tertiaire 3E (respectivement lesdites première 3E' et deuxième 3E" pièces tertiaires), est avantageusement le biocapteur décrit précédemment. Les biocapteurs formant les pièces 3E, 3E' et 3E" pourront être identiques ou différents, selon les différents modes de réalisations décrit précédemment pour le biocapteur. En particulier, l'indicateur visuel de croissance microbiologique pourra être différent ou identique d'un biocapteur à l'autre.

La composition de poudres agglomérées du biocapteur décrite précédemment sera avantageusement incorporée à la pièce tertiaire 3E (respectivement lesdites première 3E' et deuxième 3E" pièces tertiaires) à la surface, et au moins en partie dans l'épaisseur, de ladite première zone 3A (respectivement lesdites première 3A' et troisième 3A" zones). Plus précisément, ladite première zone 3A, 3A' (voire troisième zone 3A") présentant de préférence une surface « inférieure », destinée à venir en regard de ladite couche intermédiaire 4, 4' et une surface « supérieure » opposée, destinée à venir en regard de ladite couche barrière 5, 5' (ou de ladite sous-couche d'adhésion 6, 6'), la composition de poudres agglomérées du biocapteur 3E, 3E' (voire biocapteur 3E") sera avantageusement agencée au voisinage de ladite surface « supérieure » et au moins en partie dans l'épaisseur de ladite première zone 3A, 3A' (voire troisième zone 3A"). De manière encore plus préférentielle, ladite première zone 3A, 3A' (voire troisième zone 3A") étant délimitée par un contour externe fermé, ladite composition de poudres agglomérées du biocapteur 3E, 3E' (voire biocapteur 3E") sera avantageusement positionnée à distance dudit contour externe, de préférence sensiblement au centre de ladite première zone 3A, 3A' (voire troisième zone 3A"), définissant une zone 3F, 3F' (voire 3F") du biocapteur 3E, 3E' (voire biocapteur 3E") comprenant ladite composition de poudres agglomérées. Ainsi, lorsque ledit liquide atteint ladite première zone 3A, 3A' (voire troisième zone 3A"), la composition de poudres agglomérées du biocapteur 3E, 3E' (voire biocapteur 3E") est alors avantageusement entourée par ledit liquide imprégnant la première zone 3A, 3A' (voire troisième zone 3A"), de sorte que la composition de poudres agglomérées du biocapteur vient en contact avec ledit liquide de manière sensiblement homogène selon toute sa périphérie.

### Couche intermédiaire

Tel qu'illustré aux figures, le dispositif médical 1, l' de l'invention comprend avantageusement en outre une couche intermédiaire 4, 4', qui est interposée entre lesdites couches primaire 2, 2' et secondaire 3, 3'. Agencée en regard, à l'aplomb, desdites couches primaire 2, 2' et secondaire 3, 3' respectivement, ladite couche intermédiaire 4, 4' s'étend de préférence selon un plan d'extension intermédiaire sensiblement parallèle aux plans d'extension primaire et secondaire desdites couches primaire 2, 2' et secondaire 3, 3'. Dans les exemples illustrés aux figures, ladite couche intermédiaire 4, 4' est avantageusement agencée d'une part en regard, à l'aplomb et au-dessus (et avantageusement en contact surfacique direct) de ladite couche primaire 2, 2' et, d'autre part, en regard, à l'aplomb et au-dessous (et avantageusement en contact surfacique direct) de ladite couche secondaire 3, 3'.

Selon l'invention, la couche intermédiaire 4, 4' comprend elle-même au moins une première portion imperméable 4A, 4A' audit liquide (c'est-à-dire étanche à ce dernier) et au moins une première portion perméable 4B, 4B' audit liquide, cette dernière permettant le passage, à travers elle, dudit liquide en provenance de la couche primaire 2, 2' vers la couche secondaire 3, 3', tandis que ladite première portion imperméable 4A, 4A' interdit au contraire le passage du liquide à travers elle. De préférence, ladite première portion imperméable 4A, 4A' et ladite première portion perméable 4B, 4B' sont respectivement continues.

Dans le premier mode de réalisation préférentiel des figures 1, 2, 5 et 6, ladite couche intermédiaire 4 comprend de préférence, outre ladite première portion imperméable 4A et ladite première portion perméable 4B, une deuxième portion imperméable 4C, avantageusement distincte de la première portion imperméable 4A. Alternativement, dans le deuxième mode de réalisation préférentiel de la figure 3, ladite couche intermédiaire 4' comprend de préférence, outre ladite première portion imperméable 4A' et ladite première portion perméable 4B', une deuxième 4C' et une troisième 4A" portions imperméables, ainsi qu'une deuxième portion perméable 4B". De préférence, ces deuxième 4C' et troisième 4A" portions imperméables sont chacune distincte de la première portion imperméable 4A', et la deuxième portion perméable 4B" est avantageusement distincte de la première portion perméable 4B'.

De telles caractéristiques de perméabilité / imperméabilité en regard d'un liquide de nature et composition données peuvent être obtenues par toute technique connue, par exemple en modulant localement la porosité ou la solubilité dans ledit liquide du matériau formant ladite couche intermédiaire 4, 4'. Cependant, ladite première portion perméable 4B (alternativement, lesdites première 4B' et deuxième 4B" portions perméables) est préférentiellement formée par au moins une première ouverture intermédiaire 4D traversante (respectivement, par au moins une première 4D' et au moins une deuxième 4D" ouvertures intermédiaires traversantes) ménagée à travers ladite couche intermédiaire 4, 4'. Ladite première ouverture intermédiaire 4D (respectivement lesdites première 4D' et deuxième 4D" ouvertures intermédiaires) sera bien entendu dimensionnée de manière adéquate pour laisser effectivement passer ledit liquide à travers elle. Ladite première portion imperméable 4A, 4A' de la couche intermédiaire 4, 4' (et de préférence également la deuxième portion imperméable 4C, 4C', voire avantageusement aussi la troisième portion imperméable 4A") est quant à elle préférentiellement formée d'un film (ou couche), préférentiellement continu, en matériau polymère hydrophobe, par exemple de type adhésif sensible à la pression (PSA) (par exemple à base de silicone) ou en polyuréthane. Ladite couche intermédiaire 4, 4' est ainsi de conception très simple et pourra avantageusement être aisément réalisée, comme cela sera détaillé plus loin, par enduction partielle d'une face de l'une ou l'autre desdites couches primaire 2, 2' et secondaire 3, 3' à l'aide dudit adhésif sensible à la pression ou de polyuréthane. On notera par ailleurs que le recours préférentiel à un tel adhésif sensible à la pression permettra avantageusement de solidariser entre elles, grâce à ladite couche intermédiaire 4, 4' intercalaire ainsi formée, lesdites couches primaire 2, 2' et secondaire 3, 3', une fois ces dernières avantageusement agencées l'une en regard de l'autre conformément à ce qui précède.

De plus, dans le cas préférentiel, illustré aux figures, où la première zone 3A (alternativement la première zone 3A', ainsi que de préférence la troisième zone 3A") est délimitée par un contour externe fermé et est entourée de manière contiguë par ladite deuxième zone 3B, 3B', la première ouverture intermédiaire 4D (alternativement la première ouverture intermédiaire 4D', ainsi que de préférence la deuxième ouverture intermédiaire 4D") forme avantageusement une première lumière 4D (respectivement une première lumière 4D', ainsi que de préférence la deuxième lumière 4D") curviligne refermée sur elle-même. En d'autres termes, la première ouverture intermédiaire 4D (alternativement la première ouverture intermédiaire 4D', ainsi que de préférence la deuxième ouverture intermédiaire 4D") se présente, dans ce cas, sous la forme d'une première lumière 4D (respectivement sous la forme d'une première lumière 4D', ainsi que de préférence d'une deuxième lumière 4D") ou d'un « canal » de largeur prédéfinie, qui s'étend dans le plan d'extension intermédiaire de la couche intermédiaire 4, 4'. Fermé sur lui-même en ses extrémités, ce « canal » débouche de manière continue, sur toute la longueur de son profil fermé, de part et d'autre de la surface de la couche intermédiaire 4, 4'.

De manière particulièrement avantageuse, le profil curviligne fermé de cette première ouverture intermédiaire 4D, 4D' (voire également et avantageusement de la deuxième ouverture 4D") est conjugué avec le profil respectif du contour secondaire externe fermé de la première zone 3A, 3A' (voire de la troisième zone 3A") de la couche secondaire 3, 3'. Ainsi, si ledit contour secondaire externe de la première zone 3A, 3A' est par exemple circulaire ou rectangulaire, ladite première ouverture intermédiaire 4D, 4D' correspondante forme alors une première lumière 4D, 4D' annulaire ou annulaire à contours interne et externe rectangulaires (figures 2, 3 et 6). Ainsi, dans le premier mode de réalisation préférentiel des figures 2 et 6, la première ouverture intermédiaire 4D de la couche intermédiaire 4 se présente de préférence sous la forme d'une lumière 4D annulaire à contours interne et externe rectangulaires, de profil conjugué à la forme rectangulaire affectée par la première zone 3A. Dans le deuxième mode de réalisation préférentiel de la figure 3, lesdites première 4B' et une deuxième 4B" portions perméables sont respectivement formées d'une première 4D' et d'une deuxième 4D" ouvertures intermédiaires. Ces première 4D' et deuxième 4D" ouvertures intermédiaires d'une part, et lesdites première 3A' et troisième zone 3A" d'autre part, sont préférentiellement identiques entres elles, et lesdites première 4D' et deuxième 4D" ouvertures intermédiaires se présentent de préférence sous la forme d'une première 4D' et d'une deuxième 4D" lumières annulaires à contours interne et externe rectangulaires, de profils respectifs conjugués à la forme rectangulaire respectivement préférentiellement affectée par lesdites première 3A et troisième zones 3A". La largeur moyenne desdites première 4D, 4D' et deuxième 4D" lumières, c'est-à-dire la distance séparant le contour interne et le contour externe de cette dernière, est par exemple comprise entre 3 et 5 mm.

De préférence, l'épaisseur de la couche intermédiaire 4, 4' est choisie inférieure, voire très inférieure, aux épaisseurs respectives des couches primaire 2, 2' et secondaire 3, 3', de manière à garantir une bonne compacité dudit dispositif médical 1, 1'. Typiquement, l'épaisseur de la couche intermédiaire 4, 4' pourra ainsi être sensiblement comprise entre 0,05 et 0,3 mm. Selon l'épaisseur choisie de la couche intermédiaire 4, 4' et la nature même dudit liquide considéré, ladite première ouverture intermédiaire 4D, 4D' (voire également et avantageusement de la deuxième ouverture 4D") pourra éventuellement être comblée par un élément complémentaire perméable audit liquide (par exemple une pièce surfacique de textile non tissé hydrophile), de sorte à faciliter le passage dudit liquide à travers la première portion perméable 4B, 4B' (voire également et avantageusement à travers la deuxième portion perméable 4B") ainsi obtenue en formant un « pont » entre lesdites couches primaire 2, 2' et secondaire 3, 3'. Alternativement, ladite (ou lesdites) ouverture intermédiaire 4D, 4D', 4D" pourra être laissée vide, sans préjudice pour le bon passage dudit liquide, dans la mesure où lesdites couches primaire 2, 2' et secondaire 3, 3' entre lesquelles est interposée ladite couche intermédiaire 4, 4' pourront venir localement en contact ou quasi contact l'une avec l'autre au niveau de ladite première ouverture intermédiaire 4D, 4D' (voire également et avantageusement de la deuxième ouverture 4C") sous l'effet de leur poids respectif.

Lesdites couche primaire 2, 2', couche secondaire 3, 3' et couche intermédiaire 4, 4' du dispositif médical 1, l' de l'invention étant ainsi décrites en détails, la configuration respective et l'agencement relatif de ces couches 2, 3, 4, 2', 3', 4' l'une par rapport à l'autre au sein dudit dispositif médical 1, l' vont à présent être présentés plus précisément. On comprendra ainsi leurs fonctions et contributions respectives au fonctionnement du dispositif médical 1, l' de l'invention.

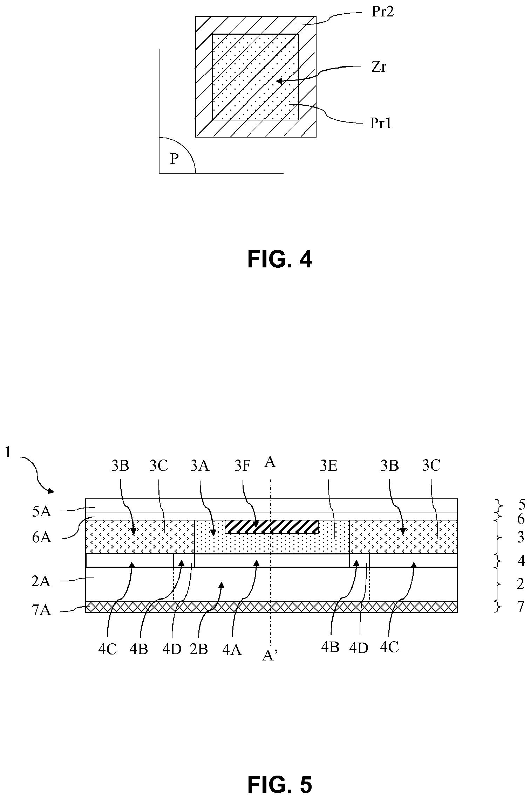

Selon l'invention, et tel qu'illustré aux figures, ladite première portion imperméable 4A, 4A' s'étend en regard de (c'est-à-dire face à), et de préférence à l'aplomb de, ladite première zone 3A, 3A' de la couche secondaire 3, 3', la projection Pr1 (orthogonale) de la première portion imperméable 4A, 4A' dans un plan P (fictif) de projection parallèle auxdites couches secondaire 3, 3' et intermédiaire 4, 4' recouvrant au moins la moitié de la surface de la projection Pr2 (orthogonale) de la première zone 3A, 3A' dans ce même plan de projection, pour limiter le reflux dudit liquide depuis la première zone 3A, 3A' de la couche secondaire 3,3' vers la couche primaire 2, 2'. Autrement dit, tel que cela ressort de la figure 4, la superficie de la zone de recouvrement Zr des projections Prl, Pr2 respectives des première portion imperméable 4A, 4A' et première zone 3A, 3A' dans le plan P est au moins égale à la moitié de la superficie de la projection Pr2 de la première zone 3A, 3A'. Cela signifie que lesdites couches secondaire 3, 3' et intermédiaire 4, 4' sont configurées et agencées l'une par rapport à l'autre au sein du dispositif médical 1, l' de manière à ce que ladite première zone 3A, 3A' fasse au moins majoritairement face à ladite première portion imperméable 4A, 4A'. Ainsi, ladite première zone 3A, 3A' et ladite première portion imperméable 4A, 4A' étant au moins majoritairement mises en correspondance selon une direction parallèle à l'axe A-A', B-B' orthogonal aux couches, ladite couche intermédiaire 4, 4' rend avantageusement au moins la moitié de la surface de la première zone 3A, 3A' inaccessible audit liquide selon une direction orthogonale aux couches secondaire 3, 3' et intermédiaire 4, 4'. On comprend dès lors que le reflux, et en particulier le reflux « direct » (c'est-à-dire selon un trajet sensiblement linéaire et orthogonal aux couches primaire 2, 2', secondaire 3, 3' et intermédiaire 4, 4'), dudit liquide depuis ladite première zone 3A, 3A' de la couche secondaire 3, 3' vers ladite couche primaire 2, 2', via la couche intermédiaire 4, 4', est ainsi particulièrement contrôlé et limité. De préférence, la projection Pr1 (orthogonale) de la première portion imperméable 4A, 4A' dans ledit plan P de projection recouvre au moins 55 %, de préférence encore au moins 60 %, et de façon encore plus préférentielle au moins 80 % de la surface de la projection Pr2 (orthogonale) de la première zone 3A, 3A' dans ce même plan P, afin d'optimiser l'effet anti-reflux recherché. Selon une variante particulièrement avantageuse, visible à la figure 6, la projection Pr1 de la première portion imperméable 4A, 4A' dans ledit plan P parallèle auxdites couches secondaire 3, 3' et intermédiaire 4, 4' est confondue avec la projection Pr2 de la première zone 3A, 3A' dans ce même plan P (recouvrement à 100 %), Ainsi, le reflux « direct » du liquide depuis la première zone 3A, 3A' de la couche secondaire 3, 3' vers la couche primaire 2, 2', via la couche intermédiaire 4, 4', est avantageusement interdit.

Dans le mode de réalisation préférentiel de la figure 3, tandis que ladite première portion imperméable 4A' s'étend en regard de (et de préférence à l'aplomb de) ladite première zone 3A', ladite troisième portion imperméable 4A" s'étend préférentiellement en regard de, et de préférence à l'aplomb de, ladite troisième zone 3A", la projection (orthogonale) de la troisième portion imperméable 4A" dans un plan (fictif) de projection parallèle auxdites couches secondaire 3' et intermédiaire 4' recouvrant au moins la moitié de la surface respective des projections (orthogonales) de la première zone 3A' et de la troisième zone 3A" dans ce même plan de projection. Compte tenu de ce qui précède, on comprend ainsi que le reflux (et en particulier le reflux « direct ») du liquide depuis chacune desdites première 3A' et troisième 3A" zones de la couche secondaire 3' vers ladite couche primaire 2', via la couche intermédiaire 4', est ainsi particulièrement contrôlé et limité. De préférence, la projection (orthogonale) de la troisième portion imperméable 4A" dans ledit plan de projection recouvre au moins 55 %, de préférence encore au moins 60 %, et de façon encore plus préférentielle 80 % de la surface de la projection (orthogonales) de la troisième zone 3A" dans ce même plan. Selon une variante (non illustrée), la projection de la troisième portion imperméable 4A" dans ledit plan de projection parallèle auxdites couches secondaire 3' et intermédiaire 4' est elle-même confondue avec la projection respective de la troisième zone 3A" dans ce même plan (recouvrement à 100 %).

Ladite couche intermédiaire 4, 4' comprend de préférence (comme évoqué ci-avant), une deuxième portion imperméable 4C, 4C' audit liquide. Avantageusement, cette deuxième portion imperméable 4C, 4C' s'étend en regard de, c'est-à-dire face à, ladite deuxième zone 3B, 3B' de la couche secondaire 3, 3', la projection (orthogonale) de la deuxième portion imperméable 4C, 4C' dans un plan (fictif) de projection parallèle auxdites couches secondaire 3, 3' et intermédiaire 4, 4' recouvrant au moins la moitié de la surface de la projection (orthogonale) de la deuxième zone 3B, 3B' dans ce même plan de projection, pour limiter ainsi avantageusement le reflux (et en particulier le reflux « direct ») dudit liquide depuis ladite deuxième zone 3B, 3B' vers ladite couche primaire 2, 2'. En d'autres termes, lesdites couches secondaire 3, 3' et intermédiaire 4, 4' sont avantageusement configurées et agencées l'une par rapport à l'autre au sein dudit dispositif médical 1, l' de manière à ce que la deuxième zone 3B, 3B' fasse au moins majoritairement face à la deuxième portion imperméable 4C, 4C'. Ainsi, la deuxième zone 3B, 3B' et la deuxième portion imperméable 4C, 4C' étant au moins majoritairement mises en correspondance selon une direction parallèle à l'axe A-A', B-B' orthogonal aux couches, ladite couche intermédiaire 4, 4' rend avantageusement au moins la moitié de la surface de la deuxième zone 3B, 3B' inaccessible audit liquide selon une direction orthogonale aux couches secondaire 3, 3' et intermédiaire 4, 4'. De préférence encore, la projection (orthogonale) de la deuxième portion imperméable 4C, 4C' dans ledit plan de projection recouvre au moins 55 %, de préférence encore au moins 60 %, voire même au moins 80 % de la surface de la projection (orthogonale) de la deuxième zone 3B, 3B' dans ce même plan, afin de procurer un effet anti-reflux suffisant.

Selon une variante, visible aux figures 1 à 3, la projection (orthogonale) de ladite première portion perméable 4B, 4B' dans un plan (fictif) de projection parallèle auxdites couches secondaire 3, 3' et intermédiaire 4, 4' chevauche, c'est-à-dire recouvre simultanément et au moins partiellement, les projections (orthogonales) respectives des première 3A, 3A' et deuxième 3B, 3B' zones dans ce même plan de projection. Autrement dit, lesdites couches secondaire 3, 3' et intermédiaire 4, 4' sont conçues et agencées l'une par rapport à l'autre au sein dudit dispositif médical 1,1' pour que ladite première portion perméable 4B, 4B' soit mise en correspondance à la fois avec une partie de la surface de la première zone 3A, 3A' et avec une partie de la surface de la deuxième zone 3B, 3B'. Tel que cela ressort des figures, ladite première portion perméable 4B, 4B' s'étend ainsi avantageusement à la fois sous et en contact direct avec une partie de la surface de la première zone 3A, 3A' et avec une partie de la surface de la deuxième zone 3B, 3B'.

Dans le mode de réalisation préférentiel de la figure 3, dans lequel ladite couche intermédiaire 4' comprend préférentiellement (comme évoqué ci-avant) de préférence une première 4B' et une deuxième 4B" portions perméables distinctes l'une de l'autre, la projection (orthogonale) respective de ces première 4B' et deuxième 4B" portions perméables dans un plan (fictif) de projection parallèle aux couches chevauche avantageusement, c'est-à-dire recouvre respectivement au moins partiellement, les projections (orthogonales) des première 3A' et deuxième 3B' zones, et les projections (orthogonales) des troisième 3A" et deuxième 3B' zones dans ce même plan de projection. Autrement dit, ladite première portion perméable 4B' s'étend avantageusement à la fois en regard de, et de préférence en contact direct avec une partie de la surface de la première zone 3A' et avec une partie de la surface de la deuxième zone 3B'. Respectivement, ladite deuxième portion perméable 4B' s'étend avantageusement à la fois en regard de, et de préférence en contact direct avec une partie de la surface de la troisième zone 3A" et avec une partie de la surface de la deuxième zone 3B'.

De manière plus préférentielle encore, , ladite première zone 3A, étant avantageusement délimitée par un contour externe fermé et étant entourée de manière contiguë par ladite deuxième zone 3B, et ladite première ouverture intermédiaire 4D formant avantageusement une première lumière 4D curviligne refermée sur elle-même, tel qu'envisagé ci-avant, la projection (orthogonale) dudit contour de la première zone 3A dans un plan (fictif) parallèle auxdites couches secondaire 2 et intermédiaire 4 se superpose de manière continue à la projection (orthogonale) de ladite première lumière 4D curviligne correspondante dans ce même plan parallèle (figure 2). En d'autres termes, lesdites couches secondaire 3 et intermédiaire 4 sont préférentiellement configurées et agencées l'une par rapport à l'autre au sein dudit dispositif médical 1 pour que la projection orthogonale, dans un plan parallèle auxdites couches, de tout point du contour de la première zone 3A est contenue dans la projection orthogonale de la lumière 4D curviligne correspondante dans ce même plan. De la sorte, ladite couche intermédiaire 4 autorise avantageusement, par le bais de ladite portion perméable 4B, le passage simultané dudit liquide depuis la couche primaire 2 vers l'une et l'autre desdites première 3A et deuxième 3B zones de la couche secondaire 3, tandis que le reflux du liquide depuis ces première 3A et deuxième 3B zones vers la couche primaire 2 est sensiblement limité par la présence de ladite portion imperméable 4A.

Une configuration en tout point similaire sera avantageusement retenue s'agissant du deuxième mode de réalisation de la figure 3. Ainsi, lesdites première 3A' et troisième 3A" zones étant avantageusement chacune délimitée par un contour externe fermé et entourée de manière contiguë par ladite deuxième zone 3B', et lesdites premières 4D' et deuxième 4D" ouvertures intermédiaires formant respectivement une première 4D' et une deuxième 4D" lumières curvilignes refermées sur elles-mêmes, la projection (orthogonale) respective du contour des première 3A' et troisième 3A" zones dans un plan (fictif) parallèle auxdites couches secondaire 2, 2' et intermédiaire 4, 4' se superpose de manière continue à la projection (orthogonale) respective des première 4D' et deuxième 4D" lumières curvilignes correspondantes dans ce même plan parallèle. De la sorte, la couche intermédiaire 4' autorise avantageusement, par le bais respectivement des première 4B' et deuxième 4B" portions perméables, le passage simultané dudit liquide depuis la couche primaire 2, 2' vers chacune des première 3A', deuxième 3B' et troisième 3B" zones de la couche secondaire 3', tandis que le reflux dudit liquide depuis ces dernières vers la couche primaire2' est sensiblement limité par la présence de ladite portion imperméable 4A'.

Les figures 5 et 6 illustrent une autre variante, particulièrement avantageuse. Dans cette variante, ladite projection Pr1 (orthogonale) de la première portion imperméable 4A dans ledit plan P (fictif) parallèle auxdites couches secondaire 3 et intermédiaire 4 est confondue avec la projection Pr2 de la première zone 3A dans ce même plan, comme cela a été envisagé plus haut. En outre, ladite première zone 3A est préférentiellement délimitée par un contour externe fermé et est entourée de manière contiguë par ladite deuxième zone 3B, ladite première ouverture intermédiaire 4D formant une première lumière 4D curviligne refermée sur lui-même. En revanche, à la différence de la variante évoquée ci-dessus, et tel qu'illustré aux figures 5 et 6, la projection de ladite première lumière 4D curviligne dans un plan parallèle auxdites couches secondaire 3 et intermédiaire 4 entoure de manière contigüe la projection respective dudit contour de la première zone 3A dans ce même plan. En d'autres termes, lesdites couches secondaire 3 et intermédiaire 4 sont préférentiellement conçues et agencées l'une par rapport à l'autre au sein dudit complexe 1 de sorte que la projection orthogonale, dans un plan parallèle auxdites couches 3, 4, de tout point du contour externe de la première zone 3A est confondue avec la projection orthogonale du contour interne de la lumière 4D curviligne correspondante dans ce même plan.

Par analogie, cette configuration particulière pourra avantageusement être transposée au mode de réalisation de la figure 3. Ainsi, lesdites première 3A' et troisième 3A" zones étant avantageusement chacune délimitée par un contour externe fermé et entourée de manière contiguë par ladite deuxième zone 3B', et lesdites premières 4D' et deuxième 4D" ouvertures intermédiaires formant respectivement une première 4D' et une deuxième 4D" lumières curvilignes refermées sur elles-mêmes, la projection (orthogonale) respective desdites première 4D' et deuxième 4D" lumières curvilignes dans un plan parallèle auxdites couches secondaire 3' et intermédiaire 4' entourera alors de manière contigüe la projection respective des contours externes respectifs des première 3A' et troisième 3A" zones dans ce même plan.

Ainsi, la première portion perméable 4B (alternativement la première portion perméable 4B', ainsi que de préférence la deuxième portion perméable 4B") sera avantageusement agencée en regard uniquement de ladite deuxième zone 3B (respectivement de ladite deuxième zone 3B').

Une configuration selon une telle variante permet d'obtenir un très bon compromis entre, d'une part, une certaine limitation de la progression du liquide depuis la couche primaire 2, 2' vers la première zone 3A, 3A' (voire également vers la troisième zone 3A") de la couche secondaire 3, 3', en raison de l'absence d'accès direct à ladite première zone 3A, 3A' et, d'autre part, une maîtrise optimale de la problématique du reflux dudit liquide depuis la première zone 3A, 3A' (voire de la troisième zone 3A") vers la couche primaire 2, 2'.

De préférence, tel qu'illustré aux figures 2 et 6, ladite portion perméable 4B de la couche intermédiaire 4 est positionnée sensiblement en regard, et de préférence à l'aplomb, de la zone préférentielle de collecte 2B. Alternativement, tel qu'illustré à la figure 3, les première 4B' et deuxième 4B" portions perméables de la couche intermédiaire 4' sont de préférence respectivement positionnées sensiblement en regard, et de préférence à l'aplomb, de la première 2B' et de la deuxième 2B" zones préférentielles de collecte. Cela signifie que la couche primaire 2, 2' et la couche intermédiaire 4, 4' sont, quant à elles, avantageusement configurées et agencées l'une par rapport de l'autre de telle sorte que la première portion perméable 4B (alternativement les première 4B' et deuxième 4B" portions perméables) et la zone préférentielle de collecte 2B (respectivement les première 2B' et deuxième 2B" zones préférentielles de collectes) sont mises en correspondance (respectivement deux à deux) selon une direction parallèle à l'axe A-A', B-B' orthogonal aux couches. Ainsi, seule la partie dudit liquide imprégnant la couche primaire 2, 2' qui se trouve au niveau de la (ou des) zone(s) préférentielle(s) de collecte 2B, 2B', 2B" de la couche primaire 2, 2' est susceptible d'être collectée et de traverser ladite couche intermédiaire 4, 4' en direction de ladite couche secondaire 3, 3'.

### couche barrière

Selon l'invention, le dispositif médical 1, l' comprend avantageusement en outre une couche barrière 5, 5' qui recouvre, de préférence intégralement, ladite couche secondaire 3, 3'. Tel qu'illustré aux figures, cette couche barrière 5, 5' est avantageusement positionnée en regard, et de préférence à l'aplomb, de la couche secondaire 3, 3', de sorte qu'elle constitue l'une des couches les plus superficielles dudit dispositif médical 1, 1'. De fait, la couche secondaire 3, 3' est donc avantageusement interposée entre ladite couche barrière 5, 5' et la couche intermédiaire 4, 4'. Cette couche barrière 5, 5' est sensiblement imperméable audit liquide, de sorte que lorsque ce dernier a atteint la couche secondaire 3, 3', en provenance de la couche primaire 2, 2' et via la couche intermédiaire 4, 4', il ne peut avantageusement fuir, s'écouler, hors du dispositif médical 1, 1' vers le milieu extérieur audit dispositif médical 1, 1' selon une direction sécante avec le plan d'extension moyen de ladite couche barrière 5, 5'. Avantageusement, ladite couche barrière 5, 5' est également imperméable à l'eau liquide (dans la mesure où ledit liquide n'est pas lui-même un liquide aqueux), afin d'empêcher tout passage d'eau depuis le milieu extérieur au dispositif médical 1, 1' vers l'intérieur de ce dernier par l'intermédiaire de ladite couche barrière 5, 5'. De préférence, ladite couche barrière 5, 5' est en revanche perméable à l'air, et en particulier au dioxygène et à la vapeur d'eau, de sorte qu'elle est avantageusement « respirante ». De préférence encore, ladite couche barrière 5, 5' est transparente à la lumière visible, c'est-à-dire que le matériau qui forme la couche barrière 5, 5' est sensiblement translucide, ce qui permet avantageusement d'observer, à travers ladite couche barrière 5, 5', l'aspect de la couche secondaire 3, 3' sous-jacente et, en particulier, du biocapteur présent dans la première zone 3A, 3A' ou troisième zone 3A" de cette dernière, à travers ladite couche barrière 5, 5'. De préférence, ladite couche barrière 5, 5' est formée d'un film barrière 5A, 5A' en matériau souple, par exemple en polyuréthane microporeux. Selon le matériau employé, l'épaisseur moyenne de la couche barrière 5, 5' pourra être sensiblement comprise entre 20 et 50 µm.

La couche barrière 5, 5' pourra être assemblée, agencée, à recouvrement sur la couche secondaire 3, 3' en contact direct avec cette dernière ou bien, éventuellement, à l'aide d'une sous-couche d'adhésion 6, 6', continue ou non, et interposée entre ladite couche secondaire 3, 3' et ladite couche barrière 5, 5'. Cette sous-couche d'adhésion 6, 6' sera préférentiellement formée d'un film 6A, 6A' mince d'un matériau adhésif (par exemple un adhésif sensible à la pression (PSA), par exemple à base de caoutchouc ou de silicone). Avantageusement, une telle sous-couche 6, 6' sera alors elle-même choisie perméable à l'air (par exemple sous la forme d'un film mince discontinu ou microperforé) et transparente, voire optionnellement imperméable audit liquide. Dans le cas préférentiel, évoqué ci-avant, où ladite couche secondaire 3, 3' n'est pas formée d'un seul tenant, mais consiste au contraire en un assemblage surfacique de pièces secondaire 3C, 3C' et tertiaires 3E, 3E', 3E" surfaciques distinctes, ladite couche barrière 5, 5' (et, de préférence, ladite sous-couche d'adhésion 6 , 6') permettra avantageusement de maintenir l'agencement relatif desdites pièces secondaire 3C, 3C' et tertiaires 3E, 3E', 3E".

Préférentiellement, la couche barrière 5, 5' s'étend latéralement de sorte à dépasser des couches sous-jacentes et à définir une zone périphérique correspondant à la partie s'étendant au-delà des autres couches. Ladite zone périphérique vient, lorsque le dispositif médical 1, 1' est positionné sur la peau, les muqueuses, directement en regard de la peau, des muqueuses. De façon préférée, la zone périphérique s'étend sur au moins deux côtés opposés du dispositif médical 1, 1', en d'autres termes s'étend de part et d'autre des autres couches. Cette zone périphérique est sur la face inférieure (du côté des autres couches) de la couche barrière avantageusement enduit, d'une manière continue ou discontinue, d'un adhésif permettant l'adhésion le dispositif médical 1, 1' sur la peau, les muqueuses et son maintien au contact de la plaie.

L'adhésif peut notamment être un adhésif sensible à la pression (PSA) ou un gel à base de silicone.

Le terme "adhésifs sensibles à la pression" (PSA, de l'anglais « *pressure sensitive adhesives* »), tel qu'il est utilisé dans le présent mémoire concerne des adhésifs qui peuvent adhérer sur une surface et en être décollés sans qu'il y ait transfert de quantités notables d'adhésif sur la surface et qui peuvent être collés de nouveau sur la même ou sur une autre surface car l'adhésif conserve une partie ou la totalité de sa pégosité et de sa force d'adhésion.

Parmi les adhésifs couramment utilisés dans les dispositifs médicaux et qui sont au contact de la peau on peut citer les adhésifs sensibles à la pression à base de silicone qui sont susceptibles d'adhérer à une surface simplement par contact ou sous l'effet d'une pression légère. Ils présentent des avantages importants par rapport aux adhésifs acryliques. En effet, non seulement les adhésifs acryliques peuvent provoquer une irritation de la peau chez certains patients, mais ils ont également tendance à augmenter l'adhérence de la peau au fil du temps, rendant délicat le repositionnement du dispositif médical. Les PSA en silicone sont idéalement adaptés aux besoins accrus des nouveaux dispositifs médicaux du fait de leur biocompatibilité et de leur perméabilité permettant la diffusion de l'oxygène, du dioxyde de carbone et de la vapeur d'eau ce qui les rend parfaitement adaptés aux applications médicales dans lesquelles une aération accrue est nécessaire. Cependant, l'adhésion à la peau doit être maintenue dans une zone de confort pour le patient afin d'éviter cette gêne sensorielle liés aux phases de décollement du dispositif médical.

Pour les dispositifs médicaux, dès lors que l'aspect contact à la peau est requis, la stérilité du matériau en contact avec la peau est un critère majeur de sécurité pour un grand nombre de dispositifs médicaux et ne peut être omis car son absence peut être lourde de conséquences.

La stérilisation par ionisation est une technique est particulièrement destinée aux matériaux thermosensibles. La stérilisation par rayons gamma (y) est largement utilisée dans le milieu médical. Un rayonnement (doses comprises entre 15 et 50 kGy) est émis par un Cobalt 60 de synthèse qui entraîne la destruction de l'ADN et de l'ARN des agents pathogènes, empêchant leur réplication et l'expression des gènes. Cette méthode présente un désavantage majeur lié au fait que certains matériaux peuvent être endommagés par ce rayonnement gamma. En effet, concernant les gels en silicone, les rayonnements ionisants utilisés pour la stérilisation ont un effet particulièrement préjudiciable notamment sur leurs propriétés d'adhésion à la peau (ou « *tack* » en anglo-saxon).

Dans une variante avantageuse de l'invention, on utilisera l'adhésif décrit dans la demande FR 17/00725 (non encore publiée, déposée le 7 juillet 2017). Cet adhésif est un adhésif silicone qui après une stérilisation par rayons gamma (y) maintient ses propriétés d'adhésion adaptées à une utilisation sur la peau.

Cet adhésif silicone Z sensible à la pression est obtenu par réticulation d'une composition silicone X comprenant :
1) de 80 à 20 parties en poids d'au moins une résine silicone A comprenant des fonctions SiOH,
2) de 20 à 80 parties en poids d'au moins un polyorganosiloxane G1 comprenant au moins deux fonctions SiOH en bout de chaine ou d'au moins un polyorganosiloxane G2 comprenant au moins deux fonctions Si-vinyle en bout de chaine et ayant une viscosité comprise entre 20000 et 600000 mPa.s lorsqu'il s'agit d'une huile silicone ou ayant une consistance à 25°C comprise entre 200 mm/10 et 2000 mm/10 lorsqu'il s'agit d'une gomme silicone, et
3) au moins un solvant S,
4) une base silicone B1 apte à réagir par des réactions d'addition lorsque le polyorganosiloxane G2 est présent, et
5) éventuellement un catalyseur de condensation C1 lorsque le polyorganosiloxane G1 est présent,
avec la condition selon laquelle la quantité de solvant S est déterminée de manière à ce que la composition silicone X contient pondéralement en extrait sec de silicone de 20 à 80 %, et de préférence de 40 à 70 %.

Les viscosités dont il est question dans le présent exposé correspondent à une grandeur de viscosité dynamique à 25°C dite "Newtonienne", c'est-à-dire la viscosité dynamique qui est mesurée, de manière connue en soi, avec un viscosimètre Brookfield à un gradient de vitesse de cisaillement suffisamment faible pour que la viscosité mesurée soit indépendante du gradient de vitesse (mesure effectuée selon la norme ASTM D445― 06, 2006). Le terme gomme est utilisé pour des composés organosiliciques présentant des viscosités classiquement supérieures à ~600000 mPa.s ce qui correspond à un poids moléculaire supérieur à 260000 g.mol⁻¹. La consistance ou pénétrabilité d'une gomme est déterminée à 25°C au moyen d'un pénétromètre de type PNR12 ou modèle équivalent permettant d'appliquer sur l'échantillon une tête cylindrique dans des conditions normalisées. La pénétrabilité d'une gomme est la profondeur exprimée en dixième de millimètres à laquelle un cylindre calibré pénètre dans l'échantillon pendant une minute. A cet effet, un échantillon de gomme est introduit dans un godet en aluminium de diamètre 40 mm et d'une hauteur de 60 mm. La tête cylindrique en bronze ou en laiton mesure 6,35 mm de diamètre et 4,76 mm de hauteur et est portée par une tige métallique de 51 mm de long et de 3mm de diamètre qui s'adapte au pénétromètre. Cette tige est lestée d'une surcharge de 100 g. Le poids total de l'ensemble est de 151,8g dont 4,3 pour la pièce cylindrique et sa tige support. Le godet contenant l'échantillon de gomme est mis dans le bain thermostaté à 25 ± 0,5 °C pendant au minimum 30mn. La mesure est effectuée en suivant la notice du constructeur. Les valeurs de la profondeur (V) en dixième de millimètre et du temps (t) en secondes pour atteindre cette profondeur sont indiquées sur l'appareil. La pénétrabilité est égale à 60V.t⁻¹ exprimée en dixièmes de millimètre par minute.

Dans un premier mode de réalisation, la composition silicone X comprend :
1) de 80 à 20 parties en poids d'au moins une résine silicone A comprenant des fonctions SiOH,
2) de 20 à 80 parties en poids d'au moins un polyorganosiloxane G1 comprenant au moins deux fonctions SiOH en bout de chaine qui est une gomme silicone ayant une consistance à 25°C comprise entre 200 mm/10 et 2000 mm/10,
3) éventuellement un catalyseur de condensation Cl, et
4) au moins un solvant S,
avec les conditions selon lesquelles :
a) le ratio pondéral gomme silicone G1/résine silicone A est compris entre 0,60 et 1,00, et de préférence compris entre 0,70 et 0,90 , et
b) la quantité de solvant est déterminée de manière à ce que la composition silicone X contient pondéralement en extrait sec de silicone de 20 à 80 %, et de préférence de 40 à 70 %.

Dans un deuxième mode de réalisation, la composition silicone X comprend :
1) de 80 à 20 parties en poids d'au moins une résine silicone A comprenant des fonctions SiOH,
2) de 20 à 80 parties en poids d'au moins un polyorganosiloxane G2 comprenant au moins deux fonctions Si-vinyle en bout de chaine et qui est une gomme silicone ayant une consistance à 25°C comprise entre 200 mm/10 et 2000 mm/10,
3) une base silicone B1 apte à réagir par des réactions d'addition comprenant :
   - au moins un organohydrogénosiloxane ayant au moins 2 fonctions SiH en une quantité suffisante pour fournir un ratio molaire SiH/Sivinyl compris entre 0.5 : 1 et 20 : 1,
   - un catalyseur de la réaction d'addition C2, et
   - éventuellement un inhibiteur de la réaction d'addition, et
4) au moins un solvant S,
   avec la condition selon laquelle la quantité de solvant S est déterminée de manière à ce que la composition silicone X contient pondéralement en extrait sec de silicone de 20 à 80 %, et de préférence de 40 à 70 %.

Dans l'un ou l'autre de ces modes de réalisation, la résine silicone A comprenant des fonctions SiOH est avantageusement choisie parmi le groupe constitué par
a) les résines silicones hydroxylées de type MQ^{(OH)} qui sont des copolymères comprenant des motifs siloxy M et Q(^{OH}) de formules suivantes :
   - M =R¹R²R³SiO_{1/2}, et
   - Q^{(OH)} = (OH)SiO_{3/2},
   - avec éventuellement la présence de motif siloxy Q = SiO_{4/2}
b) les résines silicones hydroxylées de type MD^{Vi}Q^{(OH)} qui sont des copolymères comprenant des motifs siloxy M, D^{Vi} et Q^{(OH)} de formules suivantes :
   - M =R¹R²R³SiO_{1/2},
   - D^{Vi} = (Vi)(R¹)SiO_{2/2}, et
   - Q(^{OH}) = (OH)SiO_{3/2},
   - avec éventuellement la présence de motif siloxy Q= SiO_{4/2}
c) les résines silicones hydroxylées de type MM^{Vi}Q^{(OH)} qui sont des copolymères comprenant des motifs siloxy M, M^{Vi} et Q(^{OH}) de formules suivantes :
   - M =R¹R²R3SiO_{1/2},
   - M^{Vi} = (Vi)(R¹)(R²)SiO_{2/2}, et
   - Q(^{OH}) = (OH)SiO_{3/2},
   - avec éventuellement la présence de motif siloxy Q = SiO_{4/2}
d) les résines silicones hydroxylées de type MDT^{(OH)} qui sont des copolymères comprenant des motifs siloxy M, D et T^{(OH)} de formules suivantes :
   - M =R¹R²R³SiO_{1/2},
   - D = R¹R²SiO_{2/2},
   - T^{(OH)} = (OH)R¹SiO_{2/2}, et
e) les résines silicones hydroxylées de type DT^{(OH)} qui sont des copolymères comprenant des motifs siloxy D et T^{(OH)} de formules suivantes :
   - D = R¹R²SiO_{2/2},
   - T^{(OH)} = (OH)R¹SiO_{2/2},
formules dans lesquelles le symbole Vi= un groupe vinyle, les symboles R¹, R² et R³ sont choisis indépendamment les uns des autres parmi :
- les groupes alkyles linéaires ou ramifiés ayant de 1 à 8 atomes de carbone inclus et éventuellement substitués par un ou plusieurs atomes d'halogène, et de préférence choisi parmi le groupe constitué par les groupes méthyle, éthyle, isopropyle, tertiobutyle et n-hexyle, et
- les groupes aryles ou alkylaryles ayant de 6 à 14 atomes de carbone inclus, et de préférence choisi parmi le groupe constitué par les groupes phényle, xylyle et tolyle.

L'adhésif décrit dans la demande FR 17/00725 et décrit précédemment peut également être utilisé dans les différentes couches du dispositif médical 1,1' à chaque fois qu'il est fait mention d'un adhésif sensible à la pression (PSA).

### éventuelles autres couches

De préférence, et comme, illustré aux figures, le dispositif médical 1, 1' comprend en outre une couche de transfert 7, 7' perméable audit liquide. Disposée parallèlement en regard, et de préférence à l'aplomb, de ladite couche primaire 2, 2', et de manière opposée à ladite couche intermédiaire 4, 4' par rapport à ladite couche primaire 2, 2', cette couche de transfert 7, 7' permet le passage dudit liquide en provenance du milieu extérieur audit dispositif médical vers ladite couche primaire 2, 2'. Ladite couche de transfert 7, 7' constitue de préférence une couche superficielle dudit dispositif médical 1, 1', opposée à ladite couche barrière 5, 5'. De préférence, ladite couche de transfert 7, 7' est formée d'une nappe surfacique grillagée 7A, 7A', de préférence en matériau polymère, par exemple à base de silicone (par exemple de type silicone liquide (LSR)), dont la taille moyenne des mailles confère à ladite couche de transfert 7, 7' son caractère de perméabilité audit liquide. De préférence, l'épaisseur de la couche de transfert 7, 7' est sensiblement comprise entre 0,01 et 0,5 mm.

La couche de transfert 7, 7' dudit dispositif médical 1, 1' pourra être préférentiellement formée d'une nappe grillagée 7A, 7A' en matériau polymère à base de silicone présentant un faible degré de pégosité (ou pouvoir « *tackant* » en anglo-saxon), de sorte à ce que ladite de transfert 7, 7' n'adhère sensiblement pas à la plaie (et à la peau et/ou muqueuses immédiatement environnantes) contre laquelle sera positionné ledit dispositif médical. Le matériau polymère en question sera avantageusement choisi compatible d'un point de vue sanitaire, et en particulier dermatologique. Ainsi, un tel dispositif médical est d'usage particulièrement confortable et sûr, le retrait dudit dispositif médical étant sensiblement atraumatique grâce, en particulier, à la mise en œuvre d'une telle nappe grillagée 7A, 7A'.

De manière avantageuse, ladite couche barrière 5, 5' et, de préférence également ladite sous-couche d'adhésion 6, 6', pourront avantageusement former des couches de superficie (aire) supérieure aux superficies respectives desdites couches primaire 2, 2', secondaire 3, 3', intermédiaire 4, 4' et couche de transfert 7, 7'. Cela signifie que, lesdites couches 3, 4, 5, 7, 3', 4', 5', 7' et sous-couche 6, 6' étant assemblées par empilement pour former ledit dispositif médical 1,1' multicouche, les bords respectifs desdites couche barrière 5, 5' et sous-couche d'adhésion 6, 6' s'étendront au-delà des bords respectifs des couches primaire 2, 2', secondaire 3, 3', intermédiaire 4, 4' et couche de transfert 7, 7'. De la sorte, il sera avantageusement possible de plaquer et maintenir fermement en position ledit dispositif médical 1,1' contre la peau ou la muqueuse lésée, par l'intermédiaire des bords respectifs desdites couche barrière 5, 5' et sous-couche d'adhésion 6, 6', ladite couche de transfert 7, 7' venant en contact surfacique direct avec la peau ou la muqueuse lésée.

Puisque ladite première zone 3A, 3A' (voire également et avantageusement ladite troisième zone 3A") fait avantageusement au moins majoritairement face à ladite première portion imperméable 4A, 4A', de sorte que ladite couche intermédiaire 4, 4' rend au moins la moitié de la surface de la première zone 3A, 3A' (voire également et avantageusement ladite troisième zone 3A") inaccessible audit liquide selon une direction orthogonale aux couches secondaire 3, 3' et intermédiaire 4, 4', on comprend dès lors que ledit biocapteur sera lui-même majoritairement (voire même totalement) inaccessible audit liquide selon une direction orthogonale aux couches secondaire 3, 3' et intermédiaire 4, 4'. Par conséquent, le risque de reflux (et en particulier de reflux « direct ») dudit liquide depuis le point de ladite première zone 3A, 3A' (voire également et avantageusement ladite troisième zone 3A") au niveau de laquelle est positionné ledit biocapteur 3E, 3E' (voire également et avantageusement 3E"), vers la couche primaire 2, 2', est particulièrement faible. Ceci permet d'assurer le fonctionnement optimal dudit biocapteur. En particulier, lorsque ledit biocapteur inclut une substance chimique potentiellement soluble dans ledit liquide, on évite ainsi au moins en partie l'élution et la dispersion de ladite substance en direction de ladite couche primaire 2, 2', voire vers la peau ou la muqueuse en contact de laquelle est placé le dispositif médical 1, 1'.

Avantageusement, ledit dispositif médical 1, l' pourra également comprendre une couche intercalaire supplémentaire (non illustrée) qui sera interposée entre les couches intermédiaire 4, 4' et secondaire 3, 3', et plus précisément au moins entre ladite première zone 3A, 3A' et ladite une première portion imperméable 4A, 4A' (voire alternativement ou de préférence également au moins entre ladite troisième zone 3A" et ladite troisième portion imperméable 4A"). De préférence, cette couche intercalaire supplémentaire sera formée d'un film mince en matière plastique dont la face orientée en regard de la couche secondaire 3, 3' sera préférentiellement encollée d'un matériau adhésif non susceptible de réagir avec ladite composition de poudres agglomérées décrite précédemment. Elle permettra de limiter encore le risque de reflux « direct » du liquide, tout en s'opposant à la migration de ladite substance réactive vers la couche intermédiaire 4, 4'.

La sous-couche 6, 6' pourra éventuellement être conçue et configurée pour ne s'étendre en regard que de la seule deuxième zone 3B, 3B'. On évitera ainsi tout éventuel risque de perturbation du fonctionnement dudit biocapteur par le matériau de la sous-couche 6, 6'.

Ceci étant exposé, le fonctionnement préférentiel du dispositif médical 1, 1' multicouche de l'invention va être à présent décrit succinctement, en référence aux modes de réalisation préférentiels illustrés aux figures 2, 3 et 6, de manière à bien mettre en évidence le chemin (illustré aux figures sous forme de flèches) emprunté par ledit liquide à travers les différentes couches dudit dispositif médical 1, 1' lors de son guidage dans ce dernier.

Dans un premier temps, un liquide donné est mis en contact avec la couche de transfert 7, 7'. Cette dernière étant perméable audit liquide, celui-ci progresse selon une direction moyenne sensiblement orthogonale à ladite couche de transfert 7, 7' (c'est-à-dire de préférence selon l'axe A-A', B-B' des figures 2, 3 et 6) jusqu'à atteindre la couche primaire 2, 2', qui est positionnée au-dessus et à l'aplomb de ladite couche de transfert 7, 7', en contact direct avec cette dernière. Là, ledit liquide est alors avantageusement dirigé, selon une direction sensiblement parallèle au plan d'extension primaire de la couche primaire 2, 2', grâce aux éléments barrières 2C, 2C', vers la ou les zone(s) de collecte préférentielle 2B, 2B', 2B" de la couche primaire 2, 2'. Puis, ledit liquide traverse la couche intermédiaire 4, 4', qui est agencée au-dessus et à l'aplomb de ladite couche primaire 1, 1' et en contact direct avec cette dernière, via la ou les portion(s) perméable(s) 4B, 4B', 4B" de la couche intermédiaire 4, 4', selon une direction moyenne sensiblement orthogonale à ladite couche intermédiaire 4, 4', jusqu'à atteindre la couche secondaire 3, 3', de préférence au niveau de la jonction desdites première 3A, 3A' et deuxième 3B, 3B' zones (voire également et avantageusement au niveau de la jonction desdites troisième 3A" et deuxième 3B' zones) de cette dernière. Enfin, une fois ladite couche secondaire 3, 3' atteinte, ledit liquide se déplace prioritairement et majoritairement en volume selon une direction sensiblement parallèle au plan d'extension secondaire de la couche secondaire 3, 3' vers ladite première zone 3A, 3A' (voire avantageusement également depuis la troisième zone 3A"). Optionnellement, ledit liquide se déplace alors, en particulier, depuis le contour de la première zone 3A, 3A' jusqu'audit biocapteur 3E, 3E' (voire avantageusement également depuis le contour de la troisième zone 3A" jusqu'audit biocapteur 3E").

Au fur et à mesure que ladite première zone 3A, 3A' (voire avantageusement également ladite troisième zone 3A") absorbe dudit liquide, le liquide qui continue d'affluer en provenance de la couche primaire 2, 2' atteint la couche secondaire 3, 3' et s'y déplace alors majoritairement en volume selon une direction sensiblement parallèle au plan d'extension secondaire de la couche secondaire 3, 3' vers ladite deuxième zone 3B, 3B'. Ce déplacement majoritaire du liquide depuis la couche primaire 2 vers la première zone 3A, 3A' (voire avantageusement également vers la troisième zone 3A") puis vers la deuxième zone 3B, 3B' de la couche secondaire 3, 3' est avantageusement facilité par la différence de capacité et de vitesse d'absorption respectives desdites couche primaire 2, 2', première zone 3A, 3A' et deuxième zone 3B, 3B'. En revanche, le reflux, et en particulier le reflux « direct », c'est-à-dire selon une direction moyenne sensiblement orthogonale aux couches primaire 2, 2' et secondaire 3, 3' dudit liquide depuis ladite première zone 3A, 3A' (voire avantageusement également depuis la troisième zone 3A") de la couche secondaire 3 est fortement limité du fait de la construction particulière dudit dispositif médical 1,1' ci-dessus exposée.

On notera en outre que, lorsque lesdites première 3A, 3A' (voire avantageusement également ladite troisième zone 3A") et deuxième 3B, 3B' zones sont avantageusement choisies contigües, et ladite deuxième zone 3B, 3B' entourant ladite première zone 3A, 3A' (voire avantageusement également ladite troisième zone 3A"), la présence d'une quantité plus importante de liquide dans ladite deuxième zone 3B, 3B' que dans ladite première zone 3A, 3A' (voire avantageusement également dans ladite troisième zone 3A") tend également à empêcher (ou en tout cas, à limiter fortement) tout déplacement dudit liquide de la première zone 3A, 3A' (voire avantageusement également de ladite troisième zone 3A") vers la deuxième zone 3B, 3B'.

### Procédé de fabrication

L'invention concerne également, en tant que tel, un procédé de fabrication d'un dispositif médical 1, 1', lequel dispositif médical est avantageusement conforme à la description qui en a été faite ci-dessus. Par conséquent, la description qui précède en relation avec le dispositif médical 1, 1' s'applique également au présent procédé de fabrication.

Dans le procédé de l'invention, on réalise ainsi un biocapteur comprenant une pièce en matériau absorbant et hydrophile - ayant une structure préférentiellement fibreuse - fixant, en surface et/ou dans son épaisseur, une composition de poudres agglomérées comprenant des particules d''EVA ayant une surface en partie recouverte au moins d'un sel d'acide orthophosphorique et d'un indicateur visuel de croissance microbiologique, tel que décrit précédemment.

Les particules d'EVA sont avantageusement incorporées à la pièce en matériau absorbant et hydrophile sous la forme d'une composition de poudres agglomérées.

Le procédé selon l'invention comprend donc une étape d'incorporation à une pièce en matériau absorbant et hydrophile d'une composition de poudres agglomérées comprenant des particules d'EVA ayant une surface en partie recouverte au moins d'un sel d'acide orthophosphorique et d'un indicateur visuel de croissance microbiologique.

L'incorporation d'une composition sèche dans l'épaisseur d'un substrat fibreux peut être réalisée de diverses façons, notamment par des techniques dites d'imprégnation à sec. A cet effet, une composition sèche et pulvérulente est saupoudrée sur la surface d'un substrat poreux, puis les particules de ladite composition sont mises en vibration, sous l'action d'ondes ultrasonores (cf. par exemple, FR 2 866 578) ou bien sous l'action d'un champ électrique alternatif (cf. par exemple, WO 2015/044605, WO 2010/001043 ou WO 99/22920). Ces particules pénètrent et s'enfoncent alors progressivement dans les cavités du corps poreux.

La pièce en matériau absorbant et hydrophile étant délimitée par un contour externe fermé, ladite composition de poudres agglomérées du biocapteur sera avantageusement positionnée à distance dudit contour externe, de préférence sensiblement au centre de ladite pièce en matériau absorbant et hydrophile.

Pour la fabrication d'un biocapteur selon l'invention, les techniques d'imprégnation à sec mettant en œuvre un champ électrique alternatif se sont révélées particulièrement adaptées pour permettre l'incorporation d'une poudre dans l'épaisseur d'un matériau absorbant hydrophile, ayant une structure fibreuse. De ce fait, les enseignements techniques dispensés par WO 2015/044605, WO 2010/001043 et WO 99/22920 font partie intégrante de la présente description.

Avantageusement, la quantité de composition de poudres agglomérées incorporée au biocapteur selon l'invention équivaut à une concentration comprise entre 10 mg.cm⁻³ et 100 mg.cm⁻³, préférentiellement entre 30 mg.cm⁻³ et 50 g.cm⁻³.

Une fois l'imprégnation à sec réalisée, un traitement thermique permet de rendre collant l'EVA et de fixer les particules de poudres agglomérées aux fibres du substrat absorbant. Ce traitement thermique consiste avantageusement en une opération de calandrage. Le calandrage, par la pression et la température de chauffage appliquées, permet de fixer et de retenir durablement, dans l'épaisseur de la pièce en matériau absorbant et hydrophile, les particules d'EVA avec l'(es) indicateur(s) visuel(s) de croissance microbiologique exposés à leur surface. Le calandrage améliore également la planéité de la surface de la pièce en matériau absorbant et hydrophile et accroit ses capacités de capillarité et d'absorption.

Le procédé selon l'invention comprend donc avantageusement une étape de traitement thermique, avantageusement de calandrage, d'une pièce en matériau absorbant et hydrophile incorporant la composition de poudres agglomérées décrite précédemment. Le traitement thermique, avantageusement le calandrage, se fait avantageusement à une température comprise entre 50°C et 80°C.

Dans un mode de réalisation préféré, le dispositif médical 1, 1' comprend en outre au moins une couche primaire 2, 2' destinée à venir, de préférence prioritairement, en contact avec ledit liquide.

De préférence, ledit procédé comprend une étape de fourniture ou de fabrication de ladite couche primaire 2, 2', laquelle est préférentiellement formée d'une pièce de textile 2A, 2A' non-tissé, poreuse et/ou hydrophile. Avantageusement d'un seul tenant, et préférentiellement constituée à 80 % de pulpe de bois (cellulose) et à 20 % de fibres de polyester (par exemple du PET), une telle pièce primaire 2A, 2A' de textile peut, par exemple, être obtenue par voie humide ou papetière (procédé « *wet laid* »), par incorporation de fibres de polyester dans une pâte cellulosique de pulpe de bois.

Selon l'invention, ledit procédé comprend une étape de réalisation de moyens de guidage 2C, 2C' dudit liquide vers au moins une zone préférentielle de collecte 2B, 2B', 2B" dudit liquide (identifiée(s) en pointillés aux figures 2, 3 et 6), prédéfinie, de ladite la couche primaire 2, 2', telle que celle-ci a été décrite ci-avant. De préférence, comme illustré aux figures, ladite (ou lesdites) zone(s) préférentielle(s) de collecte 2B, 2B', 2B" est choisie sensiblement localisée à distance des bords primaires définissant le contour de ladite couche primaire 2, 2', de sorte à éviter tout risque de fuite dudit liquide hors de la couche primaire 2, 2' par les bords primaires de cette dernière. De préférence, lesdits moyens de guidage 2C, 2C' sont constitués d'éléments barrières 2C, 2C' en matériau polymère hydrophobe, qui se présentent avantageusement sous la forme de « barrettes » longitudinales. Ladite opération de réalisation pourra alors dans ce cas être avantageusement réalisée par enduction (localisée) à chaud (procédé « *hotmelt* » en anglo-saxon) de ladite pièce primaire 2A, 2A' de textile avec un matériau polymère hydrophobe thermofusible, par exemple de type adhésif sensible à la pression (PSA). Une telle enduction peut être mise en œuvre, par exemple, à l'aide d'au moins une buse (ou moyen similaire) qui vient déposer sélectivement ledit matériau polymère hydrophobe, à l'état fondu, à la surface de ladite pièce primaire 2A, 2A' textile. Une fois ledit matériau polymère hydrophobe fondu déposé sur la pièce primaire 2A, 2A' textile, on le laissera avantageusement imprégner toute ou partie de l'épaisseur, et de préférence toute l'épaisseur, de ladite pièce primaire 2A, 2A' textile, avant qu'il ne refroidisse, fige et forme lesdites « barrettes » longitudinales.

Ledit dispositif médical, réalisé selon le procédé de l'invention, comprend également au moins :
- une couche secondaire 3, 3', réalisée d'un seul tenant ou non, et qui comprend elle-même des première 3A, 3A' et deuxième 3B, 3B' zones, lesdites première 3A, 3A' (voire également et avantageusement, troisième 3A") et deuxième 3B, 3B' zones étant distinctes et présentant chacune un comportement respectif différent au contact dudit liquide ;
- avantageusement, une couche intermédiaire 4, 4', qui est interposée entre lesdites couches primaire 2, 2' et secondaire 3, 3', et qui comprend elle-même au moins une première portion imperméable 4A, 4A' audit liquide et au moins une première portion perméable 4B, 4B' permettant le passage dudit liquide en provenance de la couche primaire 2, 2' vers la couche secondaire 3, 3', ladite première portion imperméable 4A, 4A' s'étendant en regard de ladite première zone 3A, 3A', la projection de la première portion imperméable 4A, 4A' dans un plan parallèle auxdites couches secondaire 3, 3' et intermédiaire 4, 4' recouvrant au moins la moitié de la surface de la projection de la première zone 3A, 3A' dans ce même plan, pour limiter le reflux dudit liquide depuis ladite première zone 3A, 3A' vers ladite couche primaire 2, 2', et
- avantageusement, une couche barrière 7, 7', recouvrant ladite couche secondaire 3, 3' et étant sensiblement imperméable audit liquide.

Avantageusement conformes à la description qui en a été respectivement faite plus haut en relation avec le dispositif médical 1, 1' de l'invention, lesdites couches primaire 2, 2', secondaire 3, 3', intermédiaire 4, 4' et barrière 7, 7' sont préférentiellement distinctes les unes des autres. En particulier, ladite couche primaire 2, 2' est préférentiellement réalisée à partir d'une pièce primaire 2A, 2A' de textile, lequel textile est préférentiellement non-tissé et hydrophile, ladite pièce primaire 2A, 2A' de textile étant avantageusement drainante.

De préférence, le procédé de l'invention comprend une opération de fabrication de ladite couche intermédiaire 4, 4', par enduction de la couche primaire 2, 2' ou de la couche secondaire 3, 3' avec un matériau polymère hydrophobe. Ce matériau polymère hydrophobe pourra, par exemple, être un adhésif sensible à la pression ou du polyuréthane. De manière préférentielle, cette opération de fabrication de la couche intermédiaire 4, 4' sera réalisée par enduction partielle d'une face de l'une ou l'autre desdites couche primaire 2, 2' et secondaire 3, 3', de manière à matérialiser au moins une portion enduite, formant ladite première portion imperméable 4A, 4A', et au moins une portion non enduite, formant quant à elle ladite première portion perméable 4B, 4B' de la couche intermédiaire 4, 4'. Selon la nature dudit matériau polymère hydrophobe retenu, ladite opération de fabrication de la couche intermédiaire 4, 4' pourra être réalisée par enduction à chaud (procédé « *hotmelt* » en anglo-saxon), éventuellement suivie d'une réticulation (par rayonnement IR, UV ou autre), ou encore par transfert.

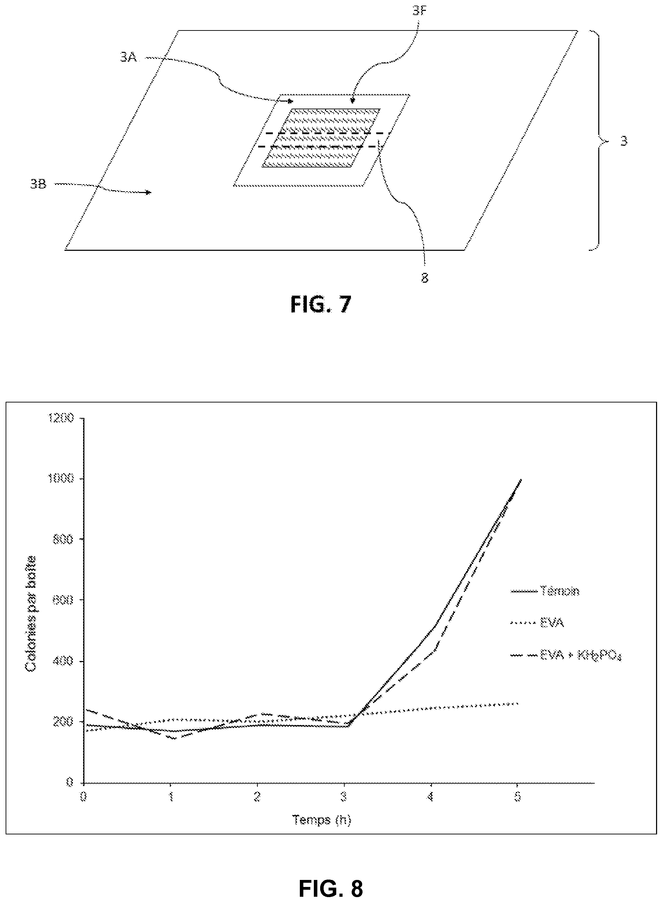

De manière particulièrement avantageuse, outre le calandrage décrit précédemment pour la réalisation du biocapteur, le procédé de l'invention pourra comprendre une opération de compression de la composition de poudres agglomérées du biocapteur 3E, 3E' et de ladite première zone 3A, 3A' de la couche secondaire 3, 3'. De préférence, ladite opération de compression est réalisée à chaud, dans des conditions de pression et de température contrôlées pour limiter notamment les risques de dégradation des propriétés de la composition de poudres agglomérées (par exemple, à une température inférieure ou égale à 80 °C). De préférence, ladite opération de compression est une opération de compression localisée dudit biocapteur 3E, 3E' et de ladite première zone 3A, 3A' de la couche secondaire 3, 3', au niveau de la zone 3F, 3F' comprenant ladite composition agglomérée, c'est-à-dire que seule une partie de ces derniers est soumise à un effort de compression, par exemple selon une ligne 8 de compression rectiligne traversant ladite zone 3F, 3F', telle que délimitée par des tirets à la figure 7. De préférence, ladite opération de compression sera réalisée avant l'assemblage de la couche secondaire 3, 3' relativement à la première couche primaire 2, 2'. Selon un mode de réalisation préférentiel du procédé selon l'invention, ladite opération de compression est avantageusement mise en œuvre à l'aide d'une plaque chauffée dont la surface est pourvue d'une zone en relief, et qui pressée contre la première zone 3A, 3A' et la zone 3F, 3F' de la couche secondaire 3, 3', de sorte à provoquer localement un écrasement de matière.

Une telle opération de compression permet avantageusement de favoriser, par capillarité, la mise en contact du liquide arrivant au niveau de la première zone 3A, 3A' avec la composition de poudres agglomérées du biocapteur 3E, 3E'.

De manière plus générale, il pourra être intéressant de créer un ou plusieurs points de compression de toute ou partie des couches du dispositif médical 1,1' entre elles, afin de favoriser le déplacement du liquide au sein de ce dernier, selon un principe similaire à celui exposé ci-dessus.

### EXEMPLES

### Exemple 1 : Démonstration de l'effet bactériostatique de l'EVA et de l'inhibition de cet effet par addition de sels d'acide orthophosphorique (KH₂PO₄, NaH₂PO₄)

### A. Démonstration réalisée sur des milieux de culture gélosés

Les effets de l'EVA et des sels de l'acide orthophosphorique (plus particulièrement, KH₂PO₄ et/ou NaH₂PO₄) sur la croissance et le développement bactériens ont été évalués, dans un premier temps, sur des milieux de culture gélosés, en l'occurrence :
- une gélose Trypto-caséine soja (TSA), un milieu universel connu comme pouvant convenir à la culture et à l'enrichissement de la plupart des bactéries, qu'elles soient aérobies ou anaérobies ;
- la gélose SAID^{®} (bioMérieux, France), un milieu de culture commercialisé pour la détection spécifique de *S. aureus,*

A cet effet, des souches de *S. aureus* provenant de la collection de la Demanderesse ont été utilisées. Avant de les inoculer aux deux géloses susmentionnées, les bactéries (100 mL d'une suspension de 10³ cfu.mL⁻¹) ont été préalablement resuspendues dans du sérum humain préparé et fourni par l'Etablissement Français du Sang (EFS), complémenté ou non en EVA et/ou en sel d'acide orthophosphorique comme suit :
- s01 : sérum (EFS, référence 4566200 / 4566201)
- s02 : sérum s01 additionné d'EVA (à 53 g.L⁻¹, de concentration finale)
- s03 : sérum s01 additionné d'EVA et de NaH₂PO₄ (respectivement à 53 g.L⁻¹ et 4,5 g.L⁻¹, de concentrations finales)
- s04 : sérum s01 additionné d'EVA et de NaH₂PO₄ (respectivement à 53 g.L-1 et 9,0 g.L⁻¹, de concentrations finales)
- s05 : sérum s01 additionné de NaH₂PO₄ (à 4,5 g.L⁻¹, de concentration finale)
- s06 : sérum s01 additionné de NaH₂PO₄ (à 9,0 g.L⁻¹, de concentration finale)
- s07 : sérum s01 additionné d'EVA et de KH₂PO₄ (respectivement à 53 g.L⁻¹ et 10,6 g.L⁻¹, de concentrations finales)
- s08 : sérum s01 additionné d'EVA et de KH₂PO₄ (respectivement à 53 g.L⁻¹ et 1,5 g.L⁻¹, de concentrations finales)
- s09 : sérum s01 additionné d'EVA et de KH₂PO₄ (respectivement à 53 g.L⁻¹ et 3,0 g.L⁻¹, de concentrations finales)
- s10 : sérum s01 additionné de KH₂PO₄ (à 1,5 g.L⁻¹, de concentration finale)
- s11 : sérum s01 additionné d'EVA, de NaH₂PO₄ et de KH₂PO₄ (respectivement à 53 g.L⁻¹, 4,5 g.L⁻¹ et 1,5 g.L⁻¹, de concentrations finales)
- s12: sérum s01 additionné d'EVA, de NaH₂PO₄ et de KH₂PO₄ (respectivement à 53 g.L⁻¹, 9,0 g.L⁻¹ et 3,0 g.L⁻¹, de concentrations finales)
- s13 : sérum s01 additionné de NaH₂PO₄ et de KH₂PO₄ (respectivement à 4,5 g.L⁻¹ et 1,5 g.L⁻¹, de concentrations finales)
- s14: sérum s01 additionné de NaH₂PO₄ et de KH₂PO₄ (respectivement à 9,0 g.L⁻¹ et 3,0 g.L⁻¹, de concentrations finales).

L'EVA utilisé dans ces essais est commercialisé par la société DAKOTA, Belgique, sous la forme d'une poudre de référence UNEX EVA (EVA T1). Sa teneur pondérale en acétate de vinyle est de 28 %.

Les résultats obtenus sont compilés dans le Tableau 1, ci-après.

**Tableau 1**

| Sérums | TSA | SAID |
|---|---|---|
| 01 | tapis bactérien | tapis bactérien |
| 02 | 1 colonie | Rien |
| 03 | > 300 colonies | > 300 colonies |
| 04 | 300 colonies | 300 colonies |
| 05 | tapis bactérien | tapis bactérien |
| 06 | tapis bactérien | tapis bactérien |
| 07 | rien | Rien |
| 08 | tapis bactérien | tapis bactérien |
| 09 | tapis bactérien | tapis bactérien |
| 10 | tapis bactérien | tapis bactérien |
| 11 | rien | Rien |
| 12 | rien | Rien |
| 13 | tapis bactérien | tapis bactérien |
| 14 | tapis bactérien | tapis bactérien |

### B. Démonstration réalisée sur un support de culture absorbant et hydrophile

L'évaluation des effets de l'EVA et des sels de l'acide orthophosphorique (plus particulièrement, KH₂PO₄) sur la croissance et le développement bactérien a été poursuivie sur un support de culture absorbant et hydrophile, incorporant de l'EVA et imbibé d'une solution nutritive comprenant, entre autres, un sel d'acide orthophosphorique.

### 1. Préparation du support de culture absorbant et hydrophile

Le support de culture absorbant et hydrophile utilisé est ici une pièce d'Airlaid^{®} provenant de la société SCA, France (référence 95NN81) et ayant un grammage annoncé de 95 g.m⁻², pour une épaisseur de 2 mm.

Dans l'épaisseur de ce matériau en fibres de cellulose ont été immobilisées des particules d'EVA dont la surface a été recouverte d'une composition pulvérulente comprenant un substrat chromogène (par exemple le 5-bromo-4-chloro-3-indolyl-*α-*glucopyranoside, le 5-bromo-4-chloro-3-indolyl-N-méthyl-*α*-glucopyranoside), de la gomme de xanthane et un agent stimulateur du métabolisme des bactéries (le MnCl₂).

La gomme de xanthane est utilisée pour augmenter quelque peu la capacité d'absorption de l'Airlaid^{®} et pour générer un gel qui facilitera l'implantation et la rétention des bactéries dans l'épaisseur du matériau fibreux. Le MnCl2 quant à lui permet de stimuler le métabolisme bactérien. Le 5-bromo-4-chloro-3-indolyl-□-glucopyranoside et le 5-bromo-4-chloro-3-indolyl-N-méthyl-□-glucopyranoside, sous l'action d'hydrolyse d'une □-glucosidase, libère des chromophores de couleur bleue/verte visibles à l'œil nu. Ces substrats chromogènes permettent ainsi de marquer les colonies bactériennes exprimant une activité □-glucosidase qui se développeraient sur ce support de culture, comme par exemple des colonies de S. aureus.

Plus précisément, les essais ont été réalisés avec des pièces d'Airlaid^{®} carrées, de 2,7 cm de côté. Ces pièces en fibres de cellulose ont été imprégnées à sec d'une composition de poudres comprenant (pour environ 100 g de composition) :
- 80 g d'EVA, avec une granulométrie moyenne de l'ordre de 60-80 µm.
- 20 g de gomme de xanthane, avec une granulométrie moyenne de l'ordre de 20-30 µm.
- 0,533 g 5-bromo-4-chloro-3-indolyl-N-méthyl-*α*-glucopyranoside et 0,3 g de 5-bromo-4-chloro-3-indolyl-*α*-glucopyranoside, avec une granulométrie moyenne de l'ordre de 20-30 µm,
- 0,027 mg de MnCl₂, avec une granulométrie moyenne de l'ordre de 20-30 µm.

Pour faciliter la manipulation de cette composition de poudres, en particulier pour obtenir une bonne répartition homogène de chaque constituant dans le support absorbant, celle-ci a été préalablement soumise à un procédé de mélange à chaud en vue d'agglomérer les particules les unes aux autres.

Grâce à un chauffage et une agitation appropriée, la surface des particules d'EVA est rendue collante et les particules des autres constituants de la composition de poudres viennent y adhérer. Le chauffage est réalisé à une température de l'ordre de 50-60°C, qui entraine le ramollissement des particules d'EVA et rend leur surface collante. Le mélange est réalisé de façon mécanique.

Cette composition de poudres ainsi assemblée à chaud renferme des particules d'EVA, plus ou moins bien individualisées, dont la surface se trouve en partie tapissée de particules de 5-bromo-4-chloro-3-indolyl-N-méthyl-*α*-glucopyranoside et 5-bromo-4-chloro-3-indolyl-*α*-glucopyranoside, de particules de gomme de xanthane et de particules de MnCl₂. Elle est ensuite transférée dans l'épaisseur d'une feuille d'Airlaid^{®} par une technique d'imprégnation à sec telle que par exemple décrite dans WO 2015/044605, WO 2010/001043 et WO 99/22920. Cette technique consiste à saupoudrer la feuille d'Airlaid^{®} de la composition de poudres et à mettre les particules de ladite composition en vibration sous l'action d'un champ électrique alternatif; les particules pénètrent et s'enfoncent alors progressivement dans les cavités du corps fibreux.

Une fois imprégnée, la feuille d'Airlaid^{®} est calandrée entre deux feuilles de papier sulfurisé, la face imprégnée étant tournée du côté du plateau chauffant. Ce calandrage est réalisé sous 4 bars de pression et à 80°C, pendant 1-2 minutes.

La feuille d'Airlaid^{®} est découpée en pièces de 2,7 cm de côtés. Chacun de ces carrés d'Airlaid^{®} renferme environ 0,06 g de poudre, renfermant environ 80 % d'EVA.

### 2. Souches bactériennes utilisées et préparation de la solution nutritive

Des souches de *Staphylococcus aureus* ont été utilisées, en l'occurrence une souche sensible à la méthicilline (MSSA) provenant de la collection de la Demanderesse. Dans le cadre de ce test, les nutriments nécessaires à la croissance et au développement de ces bactéries sont apportées par du sérum humain (provenant de l'EFS) dilué au PBS. Cette solution nutritive vise à reproduire les qualités nutritionnelles des exsudats de plaies.

Diverses compositions d'exsudats synthétiques ou reconstituées sont décrites dans la littérature scientifique et peuvent être utilisées pour reproduire le test mené par les inventeurs. Un bouillon de culture de qualités nutritionnelles faibles ou encore une eau peptonée peuvent aussi être utilisés à cette même fin.

Pour évaluer l'effet de la concentration de KH₂PO₄ sur la croissance et le développement des bactéries soumises à la présence de l'EVA (contenu ici dans l'épaisseur de la pièce d'Airlaid^{®}), le sérum dilué au PBS est additionné de KH₂PO₄ : 0 g.L⁻¹, 3 g.L⁻¹, 4 g.L⁻¹, 5 g.L⁻¹ et 6 g.L⁻¹ (en concentrations finales).

### 3. Mise en œuvre du test et résultats obtenus

100 µL d'une préparation cellulaire concentrée à 10⁶ UFC.mL⁻¹ (dans du trypton sel) sont ajoutés à 900 µL de sérum dilué au PBS, avec ou sans KH₂PO₄. Les pièces d'Airlaid^{®} sont trempées dans cette solution puis incubées en jarre pendant 17 heures à 32°C, avec un peu d'eau dans la jarre pour éviter le dessèchement.

Après incubation, les pièces de support de culture sont examinées et les constatations suivantes ont pu être faites :
- Les pièces d'Airlaid^{®} ensemencées avec des bactéries et un sérum dilué au PBS, dépourvu de KH₂PO₄ sont incolores. Aucune colonie n'y a poussé.
- Une teinte de couleur verte est observée sur les pièces ensemencées avec des bactéries et un sérum dilué au PBS complémenté avec du KH₂PO₄. Une coloration particulièrement marquée est constatée pour les concentrations de KH₂PO₄ de 3 g.L⁻¹ et 5 g.L⁻¹. Cette coloration est encore plus intense à 4 g.L⁻¹ de KH₂PO₄. A 6 g.L⁻¹ de KH₂PO₄, elle est à peine observable.

### C. Démonstration en milieu de culture liquide

L'évaluation des effets de l'EVA et des sels d'acide orthophosphorique sur la croissance et le développement bactériens a également été réalisée en milieu liquide. L'objectif est d'évaluer plus particulièrement l'impact de l'EVA et du KH₂PO₄ sur la cinétique de croissance de bactéries.

A cet effet, 1 mL d'une suspension de *Staphylococcus aureus* (300 UFC.mL⁻¹) provenant de la collection de la Demanderesse a été cultivé à 37°C dans 9 mL de différentes solutions nutritives :
- du sérum dilué au PBS,
- du sérum dilué au PBS, et comprenant 18,5 g.L-1 d'EVA,
- du sérum dilué au PBS, et comprenant 18,5 g.L-1 d'EVA et 4 g.L-1 de KH2PO4.

Après chaque heure écoulée, un comptage de cellules est effectué pour un tube de culture de chaque série. Pour ce faire, les cellules de chaque tube sont repiquées sur une boîte d'agar chromID^{®} S. aureus (bioMérieux, France). Les boîtes sont incubées 24 heures à 37°C, avant de compter les colonies formées.

Les trois cinétiques de croissance ainsi mesurées sont présentées sous forme de courbes à la Figure 8.

Ces résultats confirment ceux obtenus précédemment, sur un milieu de culture solide. L'EVA a un effet bactériostatique. Cet effet bactériostatique peut être inhibé par des sels d'acide orthophosphorique, tels que KH₂PO₄. Utiliser le KH₂PO₄ en quantité pondérale correspondant à de l'ordre de 1/5 de la quantité d'EVA, permet de contrebalancer efficacement l'effet bactériostatique de l'EVA.

### Exemple 2 : Préparation de biocapteurs selon l'invention et évaluation de leur capacité à la détection et à l'identification de bactéries

### A. Préparation et assemblage

L'exemple suivant illustre la réalisation d'un biocapteur selon l'invention, suivant un mode particulier.

Dans ce mode particulier, ledit biocapteur est préparé à partir d'une pièce réalisée en matériau fibreux absorbant hydrophile. Dans l'épaisseur de ce matériau et fixées à ses fibres, des particules EVA retiennent et exposent à leur surface un mélange pulvérulent associant :
- un sel d'acide orthophosphorique (du KH₂PO₄),
- deux substrats chromogènes d'a-glucosidase (le 5-bromo-4-chloro-3-indolyl-N-méthyl-*α*-glucopyranoside et le 5-bromo-4-chloro-3-indolyl-*α-*glucopyranoside)
- un gélifiant (de la gomme de xanthane), et
- un activateur de métabolisme bactérien (du MnCl₂).

Les particules d'EVA revêtues dudit mélange pulvérulent sont préalablement préparées en mélangeant à chaud une composition pulvérulente de particules dissociées, comprenant (pour environ 100 g de composition) :
- 80 g d'EVA,
- 20 g de gomme de xanthane
- 6,67 g de KH2PO4,
- 0,533 g de 5-bromo-4-chloro-3-indolyl-N-méthyl-□-glucopyranoside et 0,3 g de 5-bromo-4-chloro-3-indolyl-□-D-glucopyranoside, et
- 0,027 g de MnCl2.

Ce mélange pulvérulent est soumis à un procédé de mélange à chaud en vue d'obtenir une composition de poudres agglomérées, mis en œuvre dans les mêmes conditions que celles du mélange à chaud précédemment décrit.

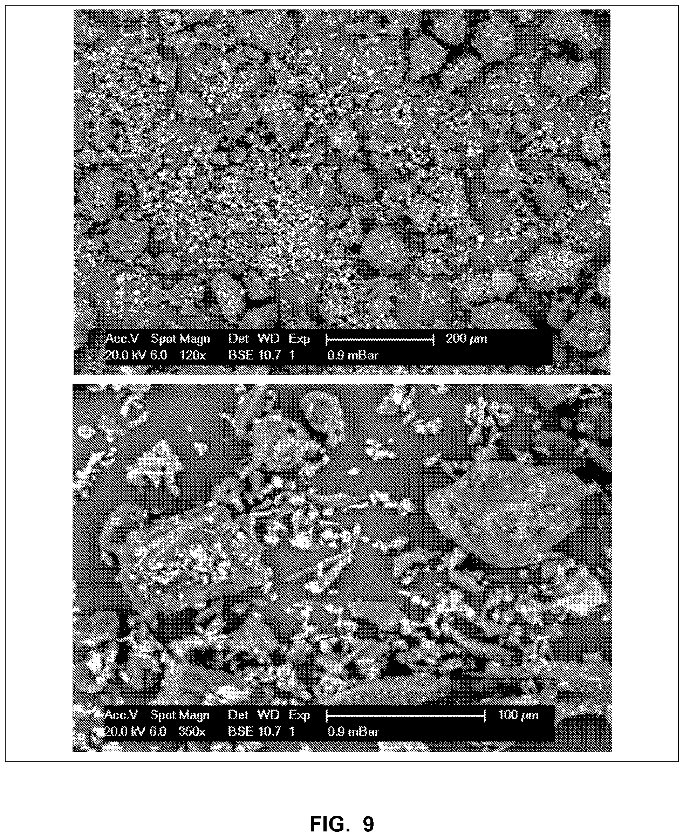

La figure 9 montre deux photographies prises en microscopie électronique à balayage de la composition pulvérulentes de particules dissociées, avant le mélange à chaud.

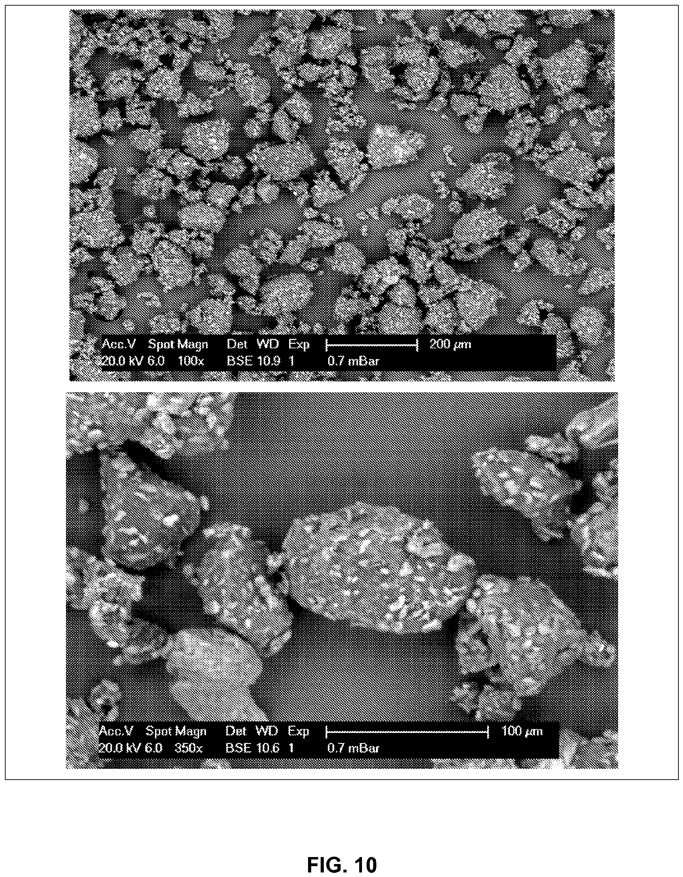

La figure 10 montre deux photographies prises en microscopie électronique à balayage d'une composition de poudres agglomérées selon l'invention, dans laquelle on distingue clairement des particules d'EVA, à la surface desquelles se répartissent de plus fines particules, en l'occurrence des particules de KH₂PO₄, de 5-bromo-4-chloro-3-indolyl-N-méthyl-*α*-glucopyranoside et 5-bromo-4-chloro-3-indolyl-*α*-glucopyranoside, de gomme de xanthane et de MnCl₂.

La composition de poudres agglomérées ainsi préparée est ensuite transférée dans l'épaisseur d'une feuille d'Airlaid^{®} par une technique d'imprégnation à sec, comme précédemment détaillé.

Une fois imprégnée, la feuille d'Airlaid^{®} est calandrée entre deux feuilles de papier sulfurisé, la face imprégnée étant tournée du côté du plateau chauffant. Ce calandrage est réalisé sous 4 bars de pression et à 80°C, pendant environ 1 minute et 30 secondes.

Des biocapteurs selon l'invention, sont découpés à partir de cette feuille d'Airlaid^{®} imprégnée à sec de la composition de poudres agglomérées, puis calandrée. Ils permettent la détection de bactéries exprimant une activité *α*-glucosidase, comme cela est le cas notamment pour les bactéries *Staphylococcus aureus.*

D'autres biocapteurs de conception très proche au premier exemple ont également été assemblés. Dans ces biocapteurs selon l'invention, les particules d'EVA exposent à leur surface également de la céfoxitine, un antibiotique à l'égard duquel les souches de *Staphylococcus aureus* sensibles à la méthicilline (MSSA) sont particulièrement sensibles, à la différence des souches résistantes à la méthicilline (MRSA). Ce deuxième exemple de biocapteurs selon l'invention permet la détection et l'identification de souches de MRSA.

La céfoxitine est fixée sur la surface des particules d'EVA de façon concomitante avec le KH₂PO₄, le 5-bromo-4-chloro-3-indolyl-N-méthyl-*α-*glucopyranoside et/ou le 5-bromo-4-chloro-3-indolyl-*α*-glucopyranoside, la gomme de xanthane et le MnCl₂, par un procédé de mélange à chaud. Pour ce faire, la composition pulvérulente de particules dissociées précédemment énoncée comprend également 6,6 mg de céfoxitine.

### B. Evaluation des biocapteurs précédents pour la détection et l'identification de Staphylococcus aureus

Des biocapteurs tels que précédemment décrits et embarquant une composition de poudres agglomérées selon l'invention, comprenant des particules d'EVA sur la surface de laquelle se répartissent :
- KH₂PO₄,
- 5-bromo-4-chloro-3-indolyl-N-méthyl-*α*-glucopyranoside
- 5-bromo-4-chloro-3-indolyl- *α*-glucopyranoside,
- gomme de xanthane et
- MnCl₂,
ont été testés pour leur aptitude à la détection de souches de *Staphylococcus aureus,* en l'occurrence des souches sensibles à la méthicilline (MSSA) provenant de la collection de la Demanderesse.

Pour ce faire, sur des carrés de biocapteurs, de 2,7 cm de côté, a été déposé 1 mL de sérum dilué au PBS, comprenant 10⁵ UFC d'une souche de MSSA (ou 1 mL de sérum dilué au PBS exempte de bactéries, pour les biocapteurs contrôles).

Les pièces d'Airlaid^{®} sont alors incubées en jarre pendant 17 heures à 32°C, avec un peu d'eau dans la jarre pour éviter le dessèchement.

Après incubation, les biocapteurs sont examinés et les constatations suivantes ont pu être faites :
- Les biocapteurs contrôles ensemencés uniquement avec du sérum dilué au PBS restent incolores.
- Une teinte de couleur verte est observée sur les biocapteurs sans céfoxitine, ensemencés avec des bactéries MSSA et le sérum dilué au PBS.
- Les biocapteurs renfermant de la céfoxitine et ensemencés avec des bactéries MSSA et le sérum dilué au PBS, restent incolores.

## Revendications

1. Dispositif médical (1, 1') non implantable destiné à recouvrir les plaies et les lésions cutanées, comprenant un biocapteur (3E, 3E'), **caractérisé en ce que** ledit biocapteur (3E, 3E') comprend une pièce en matériau absorbant et hydrophile fixant, en surface et/ou dans son épaisseur, une composition de poudres agglomérées comprenant des particules d'éthylène-acétate de vinyle (EVA) ayant une surface en partie recouverte au moins d'un sel d'acide orthophosphorique et d'un indicateur visuel de croissance microbiologique.

2. Dispositif médical (1, 1') selon la revendication 1, dans lequel le sel d'acide orthophosphorique est choisi parmi le dihydrogénophosphate de potassium (KH₂PO₄) et le dihydrogénophosphate de sodium (NaH2PO₄).

3. Dispositif médical (1, 1') selon la revendication 1 ou 2, dans lequel l'EVA et le KH₂PO₄ sont présents dans un rapport pondéral KH₂PO₄/EVA compris entre 1:25 et 1:8.

4. Dispositif médical (1, 1') selon la revendication 1 ou 2, dans lequel l'EVA et le NaH₂PO₄ sont présents dans un rapport pondéral NaH₂PO₄/EVA compris entre 1:15 et 1:3.

5. Dispositif médical (1, 1') selon l'une quelconque des revendications 1 à 4, dans lequel l'EVA présente une teneur pondérale en acétate de vinyle de 10 à 40 %.

6. Dispositif médical (1, 1') selon l'une quelconque des revendications 1 à 5 comprenant en outre une couche primaire (2, 2') adaptée pour diriger un liquide vers le biocapteur (3E, 3E').

7. Dispositif médical (1, 1') selon la revendication 6, dans lequel la couche primaire (2, 2') a une zone préférentielle de collecte (2B, 2B') dudit liquide ainsi que des moyens de guidage (2C, 2C') dudit liquide en direction de ladite zone préférentielle de collecte (2B, 2B').

8. Dispositif médical (1, 1') selon la revendication 7, comprenant en outre une couche secondaire (3, 3') intégrant ledit biocapteur (3E, 3E'), la couche secondaire (3, 3') étant disposée par rapport à la couche primaire (2, 2') de sorte à ce que ledit biocapteur (3E, 3E') s'étend en regard de la zone préférentielle de collecte (2B, 2B').

9. Dispositif médical (1, 1') selon la revendication 8, dans lequel la couche secondaire (3, 3') comprend des première (3A, 3A') et deuxième (3B, 3B') zones, lesdites première (3A, 3A') et deuxième (3B, 3B') zones étant distinctes et présentant chacune un comportement respectif différent au contact dudit liquide.

10. Dispositif médical (1, 1') selon l'une quelconque des revendications 8 à 9 comprenant en outre une couche intermédiaire (4, 4'), qui est interposée entre lesdites couches primaire (2, 2') et secondaire (3, 3').

11. Dispositif médical (1, 1') selon la revendication 10, dans lequel la couche intermédiaire (4, 4') comprend elle-même au moins une première portion imperméable (4A, 4A') audit liquide et au moins une première portion perméable (4B, 4B') permettant le passage dudit liquide en provenance de la couche primaire (2, 2') vers la couche secondaire (3, 3'), ladite première portion imperméable (4A, 4A') s'étendant en regard de ladite première zone (3A, 3A'), la projection de la première portion imperméable (4A, 4A') dans un plan parallèle auxdites couches secondaire (3, 3') et intermédiaire (4, 4') recouvrant au moins la moitié de la surface de la projection de la première zone (3A, 3A') dans ce même plan, pour limiter le reflux dudit liquide depuis ladite première zone (3A, 3A') vers ladite couche primaire (2, 2').

12. Dispositif médical (1, 1') selon l'une quelconque des revendications 8 à 11, comprenant en outre une couche barrière (5, 5'), recouvrant ladite couche secondaire (3, 3') et étant sensiblement imperméable audit liquide.

13. Dispositif médical (1, 1') selon la revendication 12, dans lequel ladite couche barrière (5, 5') est perméable à l'air, et de préférence transparente à la lumière.

14. Dispositif médical (1, 1') selon l'une quelconque des revendications 8 à 13, comprenant en outre une couche de transfert (7, 7') perméable audit liquide, disposée en regard de ladite couche primaire (2, 2') et permettant le passage dudit liquide en provenance du milieu extérieur audit dispositif médical (1, 1') vers ladite couche primaire (2, 2').

15. Dispositif médical (1, 1') selon l'une quelconque des revendications 1 à 14, qui est un pansement ou une compresse.

16. Procédé de fabrication d'un dispositif médical (1, 1') selon l'une quelconque des revendications 1 à 15, comprenant une étape d'incorporation à une pièce en matériau absorbant et hydrophile d'une composition de poudres agglomérées telle que définie à l'une quelconque des revendications 1 à 5.

17. Procédé selon la revendication 16, comprenant en outre une étape de traitement thermique, à une température comprise entre 50°C et 80°C, de la pièce en matériau absorbant et hydrophile incorporant la composition de poudres agglomérées telle que définie à l'une quelconque des revendications 1 à 5.

## Patentansprüche

1. Nicht implantierbare medizinische Vorrichtung (1, 1'), die zum Abdecken von Hautwunden und -läsionen bestimmt ist, umfassend einen Biosensor (3E, 3E'), **dadurch gekennzeichnet, dass** der genannte Biosensor (3E, 3E') ein Stück aus einem absorbierenden und hydrophilen Material umfasst, das auf der Oberfläche und/oder in seiner Dicke eine Zusammensetzung aus gepressten Pulvern fixiert, umfassend Partikel aus Äthylen-Vinylacetat (EVA) mit einer Oberfläche, die wenigstens zum Teil mit einem Salz der Orthophosphorsäure abgedeckt ist, und einen visuellen Indikator des mikrobiologischen Wachstums.

2. Medizinische Vorrichtung (1, 1') gemäß Anspruch 1, bei der das Salz der Orthophosphorsäure ausgewählt ist aus Kaliumdihydrogenphosphat (KH₂PO₄) und Natriumdihydrogenphosphat (NaH2PO₄).

3. Medizinische Vorrichtung (1, 1') gemäß Anspruch 1 oder 2, bei der das EVA und das KH₂PO₄ in einem Gewichtsverhältnis KH₂PO₄/EVA vorhanden sind, das zwischen 1:25 und 1:8 inbegriffen ist.

4. Medizinische Vorrichtung (1, 1') gemäß Anspruch 1 oder 2, bei der das EVA und das NaH₂PO₄ in einem Gewichtsverhältnis NaH₂PO₄/EVA vorhanden sind, das zwischen 1:15 und 1:3 inbegriffen ist.

5. Medizinische Vorrichtung (1, 1') gemäß irgendeinem der Ansprüche 1 bis 4, bei der das EVA einen Gewichtsanteil an Vinylacetat von 10 bis 40 % aufweist.

6. Medizinische Vorrichtung (1, 1') gemäß irgendeinem der Ansprüche 1 bis 5, umfassend darüber hinaus eine Primärschicht (2, 2'), die zum Leiten einer Flüssigkeit zum Biosensor (3E, 3E') geeignet ist.

7. Medizinische Vorrichtung (1, 1') gemäß Anspruch 6, bei der die Primärschicht (2, 2') einen bevorzugten Auffangbereich (2B, 2B') der genannten Flüssigkeit sowie Führungsmittel (2C, 2C') der genannten Flüssigkeit in Richtung des genannten bevorzugten Auffangbereichs (2B, 2B') aufweist.

8. Medizinische Vorrichtung (1, 1') gemäß Anspruch 7, umfassend darüber hinaus eine Sekundärschicht (3, 3'), die den genannten Biosensor (3E, 3E') integriert, wobei die Sekundärschicht (3, 3') in Bezug auf die Primärschicht (2, 2') derart angeordnet ist, dass der genannte Biosensor (3E, 3E') sich gegenüber dem bevorzugten Auffangbereich (2B, 2B') erstreckt.

9. Medizinische Vorrichtung (1, 1') gemäß Anspruch 8, bei der die Sekundärschicht (3, 3') erste (3A, 3A') und zweite (3B, 3B') Bereiche umfasst, wobei der genannte erste (3A, 3A') und der genannte zweite (3B, 3B') Bereich voneinander unterschiedlich sind und beim Kontakt mit der genannten Flüssigkeit jeweils ein jeweiliges unterschiedliches Verhalten aufweisen.

10. Medizinische Vorrichtung (1, 1') gemäß irgendeinem der Ansprüche 8 bis 9, umfassend darüber hinaus eine Zwischenschicht (4, 4'), die zwischen der genannten Primärschicht (2, 2') und der genannten Sekundärschicht (3, 3') zwischengeschoben ist.

11. Medizinische Vorrichtung (1, 1') gemäß Anspruch 10, bei der die Zwischenschicht (4, 4') selbst wenigstens einen ersten, für die genannte Flüssigkeit undurchlässigen Abschnitt (4A, 4A') und wenigstens einen ersten durchlässigen Abschnitt (4B, 4B') umfasst, der das Hindurchtreten der genannten Flüssigkeit von der Primärschicht (2, 2') zu der Sekundärschicht (3, 3') zulässt, wobei sich die genannte erste undurchlässige Schicht (4A, 4A') gegenüber dem genannten ersten Bereich (3A, 3A') erstreckt, wobei die Projektion der ersten undurchlässigen Schicht (4A, 4A') in einer Ebene, die parallel zu der genannten Sekundärschicht (3, 3') und der genannten Zwischenschicht (4, 4') ist, wenigstens die Hälfte der Fläche der Projektion des ersten Bereichs (3A, 3A') in dieser selben Ebene abdeckt, um den Rückfluss der genannten Flüssigkeit von dem genannten ersten Bereich (3A, 3A') zu der genannten Primärschicht (2, 2') zu begrenzen.

12. Medizinische Vorrichtung (1, 1') gemäß irgendeinem der Ansprüche 8 bis 11, umfassend darüber hinaus eine Barriereschicht (5, 5'), die die genannte Sekundärschicht (3, 3') abdeckt und für die genannte Flüssigkeit deutlich undurchlässig ist.

13. Medizinische Vorrichtung (1, 1') gemäß Anspruch 12, bei der die genannte Barriereschicht (5, 5') für Luft durchlässig und bevorzugt für Licht transparent ist.

14. Medizinische Vorrichtung (1, 1') gemäß irgendeinem der Ansprüche 8 bis 13, umfassend darüber hinaus eine Übertragungsschicht (7, 7'), die für die genannte Flüssigkeit durchlässig ist, gegenüber der genannten Primärschicht (2, 2') angeordnet ist und das Hindurchtreten der genannten Flüssigkeit von dem Außenmilieu der genannten medizinischen Vorrichtung (1, 1') zu der genannten Primärschicht (2, 2') zulässt.

15. Medizinische Vorrichtung (1, 1') gemäß irgendeinem der Ansprüche 1 bis 14, die ein Verband oder eine Kompresse ist.

16. Herstellungsverfahren einer medizinischen Vorrichtung (1, 1') gemäß irgendeinem der Ansprüche 1 bis 15, umfassend einen Einbauschritt in ein Stück aus einem absorbierenden und hydrophilen Material einer Zusammensetzung aus gepressten Pulvern gemäß Definition in irgendeinem der Ansprüche 1 bis 5.

17. Verfahren gemäß Anspruch 16, umfassend darüber hinaus einen Wärmebehandlungsschicht bei einer zwischen 50 °C und 80 °C inbegriffenen Temperatur des Stücks aus absorbierendem und hydrophilem Material, das die Zusammensetzung aus gepressten Pulvern gemäß Definition in irgendeinem der Ansprüche 1 bis 5 enthält.

## Claims

1. Non-implantable medical device (1, 1') intended to cover wounds and skin lesions, comprising a biosensor (3E, 3E'), **characterised in that** said biosensor (3E, 3E') comprises a piece made of absorbent, hydrophilic material fixing, on the surface and/or within the thickness thereof, a composition of agglomerated powders comprising particles of ethylene-vinyl acetate (EVA) having a surface in part covered with at least one orthophosphoric acid salt and a visual indicator of microbiological growth.

2. Medical device (1, 1') according to claim 1, wherein the orthophosphoric acid salt is chosen from potassium dihydrogen phosphate (KH₂PO₄) and sodium dihydrogen phosphate (NaH₂PO₄).

3. Medical device (1, 1') according to claim 1 or 2, wherein the EVA and KH₂PO₄ are present in a KH₂PO₄/EVA ratio by weight comprised between 1:25 and 1:8.

4. Medical device (1, 1') according to claim 1 or 2, wherein the EVA and NaH₂PO₄ are present in a NaH₂PO₄/EVA ratio by weight comprised between 1:15 and 1:3.

5. Medical device (1, 1') according to any of claims 1 to 4, wherein the EVA has a content by weight of vinyl acetate of 10 to 40 %.

6. Medical device (1, 1') according to any of claims 1 to 5 further comprising a primary layer (2, 2') suited to directing a liquid to the biosensor (3E, 3E').

7. Medical device (1, 1') according to claim 6, wherein the primary layer (2, 2') has a preferential collection zone (2B, 2B') of said liquid as well as means for guiding (2C, 2C') said liquid in the direction of said preferential collection zone (2B, 2B').

8. Medical device (1, 1') according to claim 7, further comprising a secondary layer (3, 3') integrating said biosensor (3E, 3E'), the secondary layer (3, 3') being arranged with respect to the primary layer (2, 2') so that said biosensor (3E, 3E') extends facing the preferential collection zone (2B, 2B').

9. Medical device (1, 1') according to claim 8, wherein the secondary layer (3, 3') comprises first (3A, 3A') and second (3B, 3B') zones, said first (3A, 3A') and second (3B, 3B') zones being distinct and each having a different respective behaviour in contact with said liquid.

10. Medical device (1, 1') according to any of claims 8 to 9 further comprising an intermediate layer (4, 4'), which is interposed between said primary (2, 2') and secondary (3, 3') layers.

11. Medical device (1, 1') according to claim 10, wherein the intermediate layer (4, 4') itself comprises at least one first portion impermeable (4A, 4A') to said liquid and at least one first permeable portion (4B, 4B') enabling the passage of said liquid coming from the primary layer (2, 2') to the secondary layer (3, 3'), said first impermeable portion (4A, 4A') extending facing said first zone (3A, 3A'), the projection of the first impermeable portion (4A, 4A') in a plane parallel to said secondary (3, 3') and intermediate (4, 4') layers covering at least half of the surface of the projection of the first zone (3A, 3A') in this same plane, to limit reflux of said liquid from said first zone (3A, 3A') to said primary layer (2, 2').

12. Medical device (1, 1') according to any of claims 8 to 11, further comprising a barrier layer (5, 5'), covering said secondary layer (3, 3') and being substantially impermeable to said liquid.

13. Medical device (1, 1') according to claim 12, wherein said barrier layer (5, 5') is permeable to air, and preferably transparent to light.

14. Medical device (1, 1') according to any of claims 8 to 13, further comprising a transfer layer (7, 7') permeable to said liquid, arranged facing said primary layer (2, 2') and enabling the passage of said liquid coming from the medium external to said medical device (1, 1') to said primary layer (2, 2').

15. Medical device (1, 1') according to any of claims 1 to 14, which is a dressing or a compress.

16. Method for manufacturing a medical device (1, 1') according to any of claims 1 to 15, comprising a step of incorporation in a piece made of absorbent, hydrophilic material of a composition of agglomerated powders such as defined in any of claims 1 to 5.

17. Method according to claim 16, further comprising a step of thermal treatment, at a temperature comprised between 50°C and 80°C, of the piece made of absorbent, hydrophilic material incorporating the composition of agglomerated powders such as defined in any of claims 1 to 5.
